# EUROPEAN PATENT APPLICATION

(11) **EP 1 097 993 A2**
(43) Date of publication of application: **09.05.2001**
(21) Application number: 00250368.8
(22) Date of filing: 03.11.2000
(51) Int. Cl.: C12N 15/11, C12N 15/62, C12N 9/00, C12N 9/90, A61K 48/00

(54) **Functional ribozyme chimeric molecules capable of sliding**

(30) Priority: 05.11.1999 JP 31613399
(71) Applicant: Secretary of Agency of Industrial Science and Technology, Tokyo 100-8921 (JP); Taira, Kazunari, Tsukuba-shi, Ibaraki 305-0046 (JP)
(72) Inventor: Taira, Kazunari, Tsukuba-shi, Ibaraki 305-0046 (JP); Warashina, Masaki, Tsukuba-shi, Ibaraki 305-0046 (JP); Kuwabara, Tomoko, Tsukuba-shi, Ibaraki 305-0046 (JP); Kawasaki, Hiroaki, Tsukuba-shi, Ibaraki 305-0005 (JP)
(74) Representative: Ziebig, Marlene, Dr. Dipl.-Chem.

(57) **Abstract**

This invention relates to a chimeric molecule comprising a region with binding affinity for a molecule capable of sliding and any functional region; a chimeric molecule comprising a molecule capable of sliding and any functional region; a chimeric molecule comprising a region with binding affinity for a molecule forming a complex with a molecule capable of sliding, and any functional region; a chimeric molecule comprising a protein capable of sliding and any functional nucleic acid; a chimeric molecule comprising a nucleic acid with binding affinity for a protein capable of sliding or a nucleic acid with binding affinity for a molecule forming a complex with said protein, and any functional nucleic acid; a vector comprising DNA encoding the chimeric molecule; and use thereof in medicaments, etc.

SEQUENCE LISTING

<110> Secretary of Agency of Industrial Science and Technology, and Kazunari
Taira

<120> Functional chimeric molecules capable of sliding

<130>

<140>
<141>

<150> JP 1999-316133
<151> 1999-11-05

<160> 53

<170> PatentIn Ver. 2.0

<210> 1
<211> 173
<212> RNA
<213> Mason-Pfizer monkey virus

<400> 1
agaccaccuc cccugcgagc uaagcuggac agccaaugac ggguaagaga gugacauugu 60
ucacuaaccu aagacaggag ggccgucaga gcuacugccu aauccaaaga cggguaaaag 120
ugauaaaaau guaucacucc aaccuaagac aggcgcagcu uccgagggat ttg 173

<210> 2

<211> 300

<212> RNA

<213> Artificial Sequence

<220>

<223> Description of Artificial Sequence: the 5' -nucleotide sequence of
LTR-luciferase chimeric gene

<400> 2

gggucucucu gguuagacca gaucugagcc ugggagcucu cuggcuaacu agggaaccca 60

cugcuuaagc cucaauaaag cuuggcauuc cgguacuguu gguaaaaugg aagacgccaa 120

aaacauaaag aaaggcccgg cgccauucua uccucuagag gauggaaccg cuggagagca 180

acugcauaag gcuaugaaga gauacgcccu gguuccugga acaauugcuu uuacagaugc 240

acauaucgag gugaacauca cguacgcgga auacuucgaa auguccguuc gguuggcaga 300

<210> 3

<211> 40

<212> RNA

<213> Artificial Sequence

<220>

<223> Description of Artificial Sequence: the nucleotide
sequence of hammerhead ribozyme (Rz)

<400> 3

nnnnnnnnnc ugaugaggcc gaaaggccga aannnnnnnn 40

<210> 4

<211> 47
<212> RNA
<213> Artificial Sequence

<220>
<223> Description of Artificial Sequence: the nucleotide
      sequence of TAR AUC Rz

<400> 4
agagcuccca ggcucacuga ugaggccgaa aggccgaaau cuggucu                47

<210> 5
<211> 40
<212> RNA
<213> Artificial Sequence

<220>
<223> Description of Artificial Sequence: the nucleotide
      sequence of LTR CUC Rz

<400> 5
agcuuuauuc ugaugaggcc gaaaggccga aaggcuuaag                        40

<210> 6
<211> 40
<212> RNA
<213> Artificial Sequence

<220>

<223> Description of Artificial Sequence: the sequence
      of Luc GUA Rz

<400> 6
uauuccgcgc ugaugaggcc gaaaggccga aacgugaugu                    40

<210> 7
<211> 40
<212> RNA
<213> Artificial Sequence

<220>
<223> Description of Artificial Sequence: the sequence
      of TAR GUU Rz

<400> 7
aucuggucuc ugaugaggcc gaaaggccga aaccagagag                    40

<210> 8
<211> 95
<212> RNA
<213> Artificial Sequence

<220>
<223> Description of Artificial Sequence: the nucleotide
      sequence of the transcript of the human placental tRNAVal

<400> 8
accguugguu uccguagugu agugguuauc acguucgccu aacacgcgaa agguccccgg   60

uucgaaaccg ggcggaaaca aagacagucg cuuuu 95

<210> 9
<211> 19
<212> RNA
<213> Artificial Sequence

<220>
<223> Description of Artificial Sequence: the sequence
of a substrate for Rz

<400> 9
nnnnnnnnun nnnnnnnnn 19

<210> 10
<211> 151
<212> DNA
<213> Artificial Sequence

<220>
<223> Description of Artificial Sequence: the sequence
encoding tRNA^val-linked TAR GUU Rz

<220>
<223> Other information: the sequence of nucleotide numbers 1-91
is of tRNA^val with 3' -modification.

<220>
<223> Other information: the sequence of nucleotide numbers 92-131

is of TAR GUU Rz.

<220>
<223> Other information: the sequence of nucleotide numbers 132-137
is of a KpnI-recognition site.

<220>
<223> Other information: the sequence of nucleotide numbers 138-140
is of a linker.

<220>
<223> Other information: the sequence of nucleotide numbers 141-146
is of an EcoRV-recognition site.

<220>
<223> Other information: the sequence of nucleotide numbers 147-151
is of a terminator.

<400> 10
accgttggtt tccgtagtgt agtggttatc acgttcgcct aacacgcgaa aggtccccgg 60
ttcgaaaccg ggcactacaa aaaccaactt tatctggtct ctgatgaggc cgaaaggccg 120
aaaccagaga gggtaccccg gatatctttt t 151

<210> 11
<211> 320
<212> DNA
<213> Artificial Sequence

<220>

<223> Description of Artificial Sequence: the sequence
encoding tRNA^val-TAR GUU Rz-CTE

<220>
<223> Other information: the sequence of nucleotide numbers 1-91 is
of tRNA^val with 3' -modification.

<220>
<223> Other information: the sequence of nucleotide numbers 92-131
is of TAR GUU Rz.

<220>
<223> Other information: the sequence of nucleotide numbers 132-137
is of a KpnI-recognition site.

<220>
<223> Other information: the sequence of nucleotide numbers 138-309
is of CTE.

<220>
<223> Other information: the sequence of nucleotide numbers 310-315
is of an EcoRV-recognition site.

<220>
<223> Other information: the sequence of nucleotide numbers 316-320
is of a terminator.

<400> 11
accgttggtt tccgtagtgt agtggttatc acgttcgcct aacacgcgaa aggtccccgg 60

ttcgaaaccg ggcactacaa aaaccaactt tatctggtct ctgatgaggc cgaaaggccg 120
aaaccagaga gggtaccaga ccacctccct gcgagctaag ctggacagcc aatgacgggt 180
aagagagtga cattgttcac taacctaaga caggagggcc gtcagagcta ctgcctaatc 240
caaagacggg taaaagtgat aaaaatgtat cactccaacc taagacaggc gcagcttccg 300
agggatttgg atatcttttt 320

<210> 12
<211> 40
<212> DNA
<213> Artificial Sequence

<220>
<223> Description of Artificial Sequence: the sequence of a probe for
Northern blotting

<400> 12
   aagatatccg ggtaccaaag ttggtttttg gtagtgcccg 40

<210> 13
<211> 40
<212> DNA
<213> Artificial Sequence

<220>
<223> Description of Artificial Sequence: the sequence of a probe for
Northern blotting

<400> 13
aagatatcca aatccctcgg aagctgcgcc tgtcttaggt 40

<210> 14
<211> 40
<212> DNA
<213> Artificial Sequence

<220>
<223> Description of Artificial Sequence: the sequence of a probe for
 Northern blotting

<400> 14
agaccagatt tcggcctttc ggcctcatca gtgagcctgg                    40

<210> 15
<211> 40
<212> DNA
<213> Artificial Sequence

<220>
<223> Description of Artificial Sequence: the sequence of a probe for
 Northern blotting

<400> 15
ctctctggtt tcggcctttc ggcctcatca gagaccagat                    40

<210> 16
<211> 40
<212> RNA
<213> Artificial Sequence

<220>

<223> Description of Artificial Sequence: the nucleotide

sequence of TAR Rz5

<400> 16

uggguucccc ugaugaggcc gaaaggccga aaguuagcca 40

<210> 17

<211> 298

<212> RNA

<213> Homo sapiens

<400> 17

cucuaaaggu ucgggggugg aauccuuggg ccgcugggca agcggcgaga ccuggccagg 60

gccagcgagc cgaggacaga gggcgcacgg agggccgggc cgcagccccg gccgcuugca 120

gaccccgcca uggacccguu ccuggugcug cugcacucgg ugucguccag ccugucgagc 180

agcgagcuga ccgagcucaa guuccuaugc cucgggcgcg ugggcaagcg caagcuggag 240

cgcgugcaga gcggccuaga ccucuucucc augcugcugg agcagaacga ccuggagc 298

<210> 18

<211> 40

<212> RNA

<213> Artificial Sequence

<220>

<223> Description of Artificial Sequence: the nucleotide

sequence of FADD-Rz1

<400> 18
gacgacaccc ugaugaggcc gaaaggccga aagugcagcu                                40

<210> 19
<211> 40
<212> RNA
<213> Artificial Sequence

<220>
<223> Description of Artificial Sequence: the nucleotide
      sequence of FADD-Rz2

<400> 19
gacaggcugc ugaugaggcc gaaaggccga aacgacaccg                                40

<210> 20
<211> 40
<212> RNA
<213> Artificial Sequence

<220>
<223> Description of Artificial Sequence: the nucleotide
      sequence of FADD-Rz3

<400> 20
ucgcugcucc ugaugaggcc gaaaggccga aacaggcugg                                40

<210> 21
<211> 40

<212> RNA
<213> Artificial Sequence

<220>
<223> Description of Artificial Sequence: the nucleotide
      sequence of FADD-Rz4

<400> 21
cauggagaac ugaugaggcc gaaaggccga aaggucuagg                                40

<210> 22
<211> 26
<212> RNA
<213> Human immunodefiency virus

<400> 22
agaccagauc ugagccuggg agcucu                                               26

<210> 23
<211> 19
<212> RNA
<213> Human immunodefiency virus

<400> 23
cuuaagccuc aauaaagcu                                                       19

<210> 24
<211> 19
<212> RNA

<213> Human immunodefiency virus

<400> 24
acaucacgua cgcggaaua 19

<210> 25
<211> 19
<212> RNA
<213> Human immunodefiency virus

<400> 25
cucucugguu agaccagau 19

<210> 26
<211> 19
<212> RNA
<213> Human immunodefiency virus

<400> 26
uggcuaacua gggaaccca 19

<210> 27
<211> 19
<212> RNA
<213> Homo sapiens

<400> 27
agcugcacuc ggugucguc 19

<210> 28

<211> 19

<212> RNA

<213> Homo sapiens

<400> 28

cggugucguc cagccuguc 19

<210> 29

<211> 19

<212> RNA

<213> Homo sapiens

<400> 29

ccagccuguc gagcagcga 19

<210> 30

<211> 19

<212> RNA

<213> Homo sapiens

<400> 30

ccuagaccuc uucuccaug 19

<210> 31

<211> 21

<212> DNA

<213> Homo sapiens

<400> 31
   aactttttgt ataaagtttg c                                                    21

<210> 32
<211> 23
<212> DNA
<213> Homo sapiens

<400> 32
ctgaagacga gtcccctggg cag                                                 23

<210> 33
<211> 23
<212> DNA
<213> Homo sapiens

<400> 33
gggctctcag gtcctgccag atg                                                 23

<210> 34
<211> 23
<212> DNA
<213> Mouse

<400> 34
tgatgatgta gtaaggtttt tgg                                                 23

<210> 35
<211> 40

<212> RNA
<213> Artificial Sequence

<220>
<223> Description of Artificial Sequence: the nucleotide
      sequence of FLASH-Rz

<400> 35
gcaaacuuua cugaugaggc cgaaaggccg aaacaaaaag                                40

<210> 36
<211> 39
<212> RNA
<213> Artificial Sequence

<220>
<223> Description of Artificial Sequence: the nucleotide
      sequence of Caspase 9-Rz

<400> 36
ccagggcuga ugaggccgaa aggccgaaac ucgucuuca                                 39

<210> 37
<211> 42
<212> RNA
<213> Artificial Sequence

<220>
<223> Description of Artificial Sequence: the nucleotide

sequence of FADD-Rz

<400> 37
caucuggcag cugaugaggc cgaaaggccg aaaccugaga gc                42

<210> 38
<211> 42
<212> RNA
<213> Artificial Sequence

<220>
<223> Description of Artificial Sequence: the nucleotide
      sequence of PTEN-Rz

<400> 38
aaaaaccucu gaugaggccg aaaggccgaa acuacaucau ca                42

<210> 39
<211> 26
<212> RNA
<213> Mouse

<400> 39
caaagcgcag uguccugcgg cgggga                                  26

<210> 40
<211> 21
<212> RNA
<213> Mouse

<400> 40
ucaguggauu caaaauccau u                                                21

<210> 41
<211> 22
<212> RNA
<213> Mouse

<400> 41
gggagcaagu caguggacuc ug                                               22

<210> 42
<211> 21
<212> RNA
<213> Mouse

<400> 42
ucuggacagu aguuacaaaa u                                                21

<210> 43
<211> 21
<212> RNA
<213> Mouse

<400> 43
acuggaaugu caucucgcuc u                                                21

<210> 44

<211> 49

<212> RNA

<213> Artificial Sequence

<220>

<223> Description of Artificial Sequence: the nucleotide
      sequence of CPP-Rz6

<400> 44

uccccgccgc agcucugaug aggccgaaag gccgaaacac ugcgcuuug                49

<210> 45

<211> 42

<212> RNA

<213> Artificial Sequence

<220>

<223> Description of Artificial Sequence: the nucleotide
      sequence of CPP-Rz7

<400> 45

aauggauuuu cugaugaggc cgaaaggccg aaaauccacu ga                42

<210> 46

<211> 43

<212> RNA

<213> Artificial Sequence

<220>

<223> Description of Artificial Sequence: the nucleotide
      sequence of CPP-Rz8

<400> 46
cagaguccac ucugaugagg ccgaaaggcc gaaacuugcu ccc                    43

<210> 47
<211> 42
<212> RNA
<213> Artificial Sequence

<220>
<223> Description of Artificial Sequence: the nucleotide
      sequence of CPP-Rz9

<400> 47
auuuuguaac cugaugaggc cgaaaggccg aaacugucca ga                    42

<210> 48
<211> 42
<212> RNA
<213> Artificial Sequence

<220>
<223> Description of Artificial Sequence: the nucleotide
      sequence of CPP-Rz10

<400> 48
agagcgagau cugaugaggc cgaaaggccg aaacauucca gu                    42

<210> 49

<211> 142

<212> RNA

<213> Artificial Sequence

<220>

<223> Description of Artificial Sequence: the nucleotide
      sequence of CPP Rz1

<400> 49

accguugguu uccguagugu agugguuauc acguucgccu aacacgcgaa agguccccgg 60

uucgaaaccg ggcacuacaa aaaccaacuu ucuagaccgc cgcagcugau gaggccgaaa 120

ggccgaaaca cugcgcggua cc 142

<210> 50

<211> 142

<212> RNA

<213> Artificial Sequence

<220>

<223> Description of Artificial Sequence: the nucleotide
      sequence of CPP Rz2

<400> 50

accguugguu uccguagugu agugguuauc acguucgccu aacacgcgaa agguccccgg 60

uucgaaaccg ggcacuacaa aaaccaacuu ucuagaaugg auuuucugau gaggccgaaa 120

ggccgaaaau ccacugggua cc 142

<210> 51

<211> 142

<212> RNA

<213> Artificial Sequence

<220>

<223> Description of Artificial Sequence: the nucleotide

sequence of CPP Rz3

<400> 51

accguugguu uccguagugu agugguuauc acguucgccu aacacgcgaa agguccccgg 60

uucgaaaccg ggcacuacaa aaaccaacuu ucuagaaagu ccacucugau gaggccgaaa 120

ggccgaaacu ugcuccggua cc 142

<210> 52

<211> 141

<212> RNA

<213> Artificial Sequence

<220>

<223> Description of Artificial Sequence: the nucleotide

sequence of CPP Rz4

<400> 52

accguugguu uccguagugu agugguuauc acguucgccu aacacgcgaa agguccccgg 60

uucgaaaccg ggcacuacaa aaaccaacuu ucuagauuug uaaccugaug aggccgaaag 120

gccgaaacug uccaggguac c 141

<210> 53

<211> 142

<212> RNA

<213> Artificial Sequence

<220>

<223> Description of Artificial Sequence: the nucleotide
      sequence of CPP Rz5

<400> 53

accguugguu uccguagugu agugguuauc acguucgccu aacacgcgaa agguccccgg 60

uucgaaaccg ggcacuacaa aaaccaacuu ucuagagagc gagaucugau gaggccgaaa 120

ggccgaaaca uuccagggua cc 142

## Description

### Technical Field of the Invention

The present invention relates to a functional chimeric molecule capable of sliding. More specifically, the present invention relates to a chimeric molecule comprising a region with binding affinity for a molecule capable of sliding or a molecule forming a complex with the molecule capable of sliding and any functional region.

In addition, the present invention relates to an expression vector comprising the chimeric molecule or DNA encoding this chimeric molecule.

In addition, the present invention relates to a complex comprising a molecule capable of sliding and a chimeric molecule.

Furthermore, the present invention relates to a pharmaceutical composition comprising the chimeric molecule, complex or expression vector as an active ingredient.

Furthermore, the present invention relates to a method of cleaving a target nucleic acid using the chimeric molecule, complex or expression vector, or a method of analyzing a biological function of the target nucleic acid.

### Background of the Invention

RNA with catalytic function, which was discovered by Cech and Altman in the 1980s, is generally called ribozyme (ribonucleic acid + enzyme). Since the discovery of ribozyme, various types of ribozymes were found, and the significance of RNA as a molecule has been brought into focus in view of organic evolution. On the other hand, as a result of the artificial development and improvement of ribozymes, it has now become possible to cleave any RNA strand site-specifically with an RNA comprising only approx. 30 residues. (O.C. Uhlenbech (1987) *Nature* 328:596-600; J. Haseloff and W.L. Gerlach (1988) *Nature* 334:585-591; F. Eckstein and D.M.J. Lilley (eds) (1996) *Catalytic RNA*, *Nucleic Acids and Molecular Biology* Vol. 10, Springer-Verlag Press etc.) Since ribozyme has high substrate specificity, it does not act nonspecifically in a cell as distinct from azidothymidine, thionylated-antisense DNA and the like. For this reason, the applied research into ribozymes for gene therapy targeting cancers or AIDS has actively been conducting throughout the world (F. Eckstein and D.M.J. Lilley, (eds) (1996) *Catalytic RNA*, *Nucleic Acids and Molecular Biology* Vol. 10, Springer-Verlag Pressp; N. Sarver *et al*., (1990) *Science* 247:1222-1225; J. Ohkawa *et al*., (1993) *Proc. Natl. Acad. Sci. USA* 90:11302-11306; S. Altman (1993) *Proc. Natl. Acad. Sci. USA* 90:10898-10900 etc.) Furthermore, a method of using a ribozyme to control the expression of a specific target gene is very effective in study toward clarifying the function of a genetic product which has an unknown action and searching interaction in a cell, and so this method has achieved great success even in basic researches in molecular biology (H. Kawasaki *et al*., (1998) *Nature* 393:284-289 etc.)

Further, presently studies for utilizing not only ribozyme but also functional nucleic acids including antisense, aptamer and so on have also been conducting throughout the world.

Hammerhead ribozyme (Rz) is one of the smallest catalytic RNAs (Symons, R. H. *Ann. Rev. Biochem*., **61**, 641-671 (1992)). They have been tested as potential therapeutic agents and their mechanisms of action have been studied (Symons, R. H. *Ann. Rev. Biochem*., **61**, 641-671 (1992) ; Zhou, D.-M. & Taira, K. *Chem. Rev*. **98**, 991-1026 (1998) ; Eckstein F. & Lilley D. M. J. (eds.) Catalytic RNA, Nucleic Acids and Molecular Biology, vol. 10 (Springer-Verlag, Berlin, 1996)). These RNAs can cleave oligoribonucleotides at specific sites (namely, after the sequence NUX, where N and X can be A, G, C or U and A, C or U, respectively, with the most efficient cleavage occurring after a GUC triplet) (Shimayama, T., Nishikawa, S. & Taira, K. *Biochemistry* **34**, 3649-3654 (1995)). Thus, RNA molecules consisting of only about thirty nucleotides can be generated for use as artificial endonucleases that can cleave specific RNA molecules (Haseloff, J. & Gerlach, W. L. *Nature* **334**, 585-591 (1988)). To date, numerous studies directed towards the application of ribozymes *in vivo* have been performed and many successful experiments, aimed at the exploitation of ribozymes for the suppression of gene expression in different organisms, have been reported (Sullenger, B. A. & Cech, T. R. *Science* **262**, 1566-1569 (1993) ; Yu, M., *et al*. *Proc. Natl. Acad. Sci. USA* **92**, 699-703 (1995) ; Bertrand, E. *et al. RNA* **3**, 75-88 (1997); Kawasaki, H., *et al. Nature* **393**, 284-289 (1998) ; Kuwabara, T., *et al. Nature Biotechnol*. **16**, 961-965 (1998) ; Kuwabara, T., *et al. Mol. Cell* **2**, 617-627 (1998); Plehn-Dujowich, D. & Altman, *S. Proc. Natl. Sci. Acad. USA* **95**, 7327-7332 (1998); Koseki, S., *et al. J. Virol.* **73**, 1868-1877 (1999) ; Tanabe, T. *et al. Nature* **406**, 473-474 (2000)).

Achieving the kind of excellent results such as these expected of gene therapy depends on whether or not a functional nucleic acid has activity within a cell. In a cell, mRNA has an intertwined higher-order structure so that complementary base pairs (stem structure) can be formed in various parts of single-stranded mRNA. It can be said that, likening mRNA to a single thread, it would have a higher-order structure wherein the thread intertwines in some places. If a cleavage site of ribozyme existed in this intertwined thread (e.g. mRNA), there would be a much lower probability of binding ribozyme and the cleavage site. For this reason, there is a problem that the majority of rybozymes whose activities were confirmed *in vitro* can not function effectively *in vivo*. The prediction about the secondary structure of a target mRNA by a computer is not necessarily consistent with the actual higher-order structure of mRNA. So, it often happens that an effective ribozyme can not be obtained through assay, despite that one has tried to construct it spending much time. Though a large number of studies for detecting the sites to which antisense can bind easily by calculating and predicting the higher-order structure of mRNA have been done, the establishment of a general approach corresponding to any target has not been successful so far.

Under the circumstances, by conferring a high affinity for target nucleic acid, a sliding function and a function of unwinding the higher-order structure of a nucleic acid to functional nucleic acids including ribozyme, the present inventors have succeeded in creating a highly functional nucleic acid, which can effectively detect a target and act on it, even if the target exists inside of intertwined complicated structure of nucleic acid.

One object of the present invention is to provide a chimeric molecule comprising a region with binding affinity for a molecule capable of sliding or a region with binding affinity for a molecule forming a complex with the molecule capable of sliding and any functional region, or a chimeric molecule comprising a molecule capable of sliding and any functional region. Such regions constituting a chimeric molecule include any of the high molecular region and low molecular region, the high molecular region and high molecular region, the low molecular region and high molecular region, and the low molecular region and low molecular region.

Another object of the present invention is to provide an expression vector comprising the chimeric molecule or DNA encoding this chimeric molecule.

A further object of the present invention is to provide a complex of the chimeric molecule and a molecule capable of sliding.

Another object of the present invention is to provide a pharmaceutical composition comprising the chimeric molecule, complex or expression vector as an active ingredient.

A further object of the present invention is to provide a method of specifically cleaving a target nucleic acid using the chimeric molecule, complex or expression vector.

Another object of the present invention is to provide a method of analyzing the biological function of a target nucleic acid using the chimeric molecule, complex or expression vector.

### Summary of the Invention

The summary of the present invention is as follows.

The present invention provides a chimeric molecule comprising a region with binding affinity for a molecule capable of sliding and any functional region.

The present invention also provides a chimeric molecule comprising a molecule capable of sliding and any functional region.

Furthermore, the present invention provides a chimeric molecule comprising a region with binding affinity for a molecule forming a complex with a molecule capable of sliding and any functional region. Herein, the molecules forming a complex with a molecule capable of sliding include, for example, adapter molecules.

The chimeric molecules of the present invention may be, for example, a nucleic acid, a peptide nucleic acid, a protein or a combination thereof. The functional regions include, for example, one having enzyme or catalytic function, or one having inhibitory function or promoting function.

In one embodiment of the present invention, the region having binding affinity for a molecule capable of sliding or the region having binding affinity for a molecule forming a complex with a molecule capable of sliding is a nucleic acid. When the molecules capable of sliding are proteins, the proteins include DNA binding protein, RNA binding protein and the like. For example, such proteins include, but are not limited to, (DNA or RNA) helicase, restriction enzyme, (DNA or RNA) polymerase, repressor etc.

In another embodiment of the present invention, the functional region is a functional nucleic acid selected from the group consisting of a ribozyme, a DNA enzyme, an antisense RNA, an antisense DNA and an aptamer. As a ribozyme, hammerhead ribozyme is preferable. Or, the functional regions include functional proteins such as restriction enzyme and antibody, or any substance such as physiologically active substance and agent.

More specifically, the chimeric molecule of the present invention comprises a nucleic acid with binding affinity for a protein capable of sliding or a nucleic acid with binding affinity for a molecule forming a complex with the protein, and any functional nucleic acid.

In the present invention, a region with binding affinity for a molecule capable of sliding or a region with binding affinity for a molecule forming a complex with the molecule capable of sliding binds directly or indirectly to the functional region. When it binds indirectly, a linker or adapter whose length is suitable for the distance between the regions may exist.

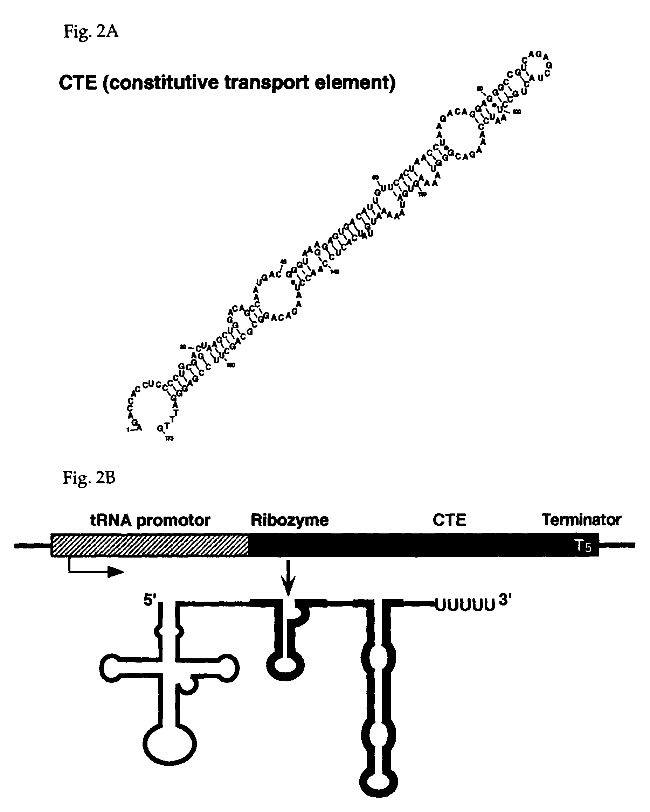

In another embodiment of the present invention, a region with binding affinity for the protein capable of sliding or a region with binding affinity for a molecule forming a complex with the protein is a nucleic acid with binding affinity for helicase or a protein forming a complex with the helicase. Examples of such nucleic acids include CTE (constitutive transport element) with binding affinity for RNA helicase or a protein forming a complex with the RNA helicase or a nucleic acid having substantially equivalent functions to the CTE, or a nucleic acid having poly(A) sequence. An example of nucleic acids having substantially equivalent functions to the CTE is a nucleic acid molecule (RNA or DNA) artificially synthesized by *in vitro* selection (SELEX). Specifically, CTE consists of a sequence shown in Figure 2A (or SEQ ID NO: 1) or a variant having substantially equivalent functions to the CTE.

In another embodiments of the present invention, it provides an expression vector comprising DNA encoding the chimeric molecule (in the case of DNA) or a chimeric molecule (in the case of RNA or protein).

In general, DNA encoding the chimeric molecule or the above chimeric molecule is controlled by a promoter. Examples of promoters include polymerase III promoter such as tRNA promoter, particularly tRNA^{val} promoter or a variant thereof. An example of the variant is a tRNA^{val} promoter wherein a bulge structure is introduced to the region where hydrogen bonds are formed between nucleotides 8-14 and nucleotides 73-79 of the RNA nucleotide sequence having secondary structure (I) set forth below.

5'-a    a g u c g c u u u u-3'
c    c
c    a
g    g
u    a
u    a
g    A
G - C
U - A
U - A
U - A
C - G
C - G
U  G - C  G G G C C A A A
A    | | | | | | | |    G
U G  A U G U G    C C C G G U U C
G    | |  | | |    C U
G U U A  C A C    G
U    G
G  U - A  A G
U - A
C - G
G - C
C - G
C    C
U    A
A A C
(I)

The bulge structure as used herein refers to a part wherein a double-stranded structure bulges because base pairs cannot be formed.

The expression vector of the present invention can further comprise a terminator sequence downstream of the chimeric molecule.

In one embodiment of the present invention, the chimeric molecule comprises a functional RNA sequence and CTE sequence. The functional RNA sequence may be selected from the group consisting of a ribozyme sequence, an antisense RNA sequence and an aptamer sequence, but is not limited thereto. In Examples described later, ribozyme is used as a functional nucleic acid, but the present invention intends to comprise any chimeric molecule including any functional region (or any functional molecule) upon which a sliding function is conferred.

In another embodiment of the present invention, there is provided a method of producing the chimeric molecule, including a process of synthesizing RNA using the expression vector DNA as a template and collecting the generated RNA.

In another embodiment of the present invention, there is provided a complex of the chimeric molecule and the molecule capable of sliding. Examples of the molecule capable of sliding include helicase such as RNA helicase and DNA helicase, and other proteins (e.g. restriction enzyme, polymerase, repressor etc.) In Examples of the present invention, helicase is used as the molecule capable of sliding, but the present invention is not limited thereto. Helicase can bind to a chimeric molecule via an adapter.

In another embodiment of the present invention, there is provided a pharmaceutical composition comprising the chimeric molecule, complex or expression vector as an active ingredient.

An example of the pharmaceutical composition is one preventing or treating viral diseases, diseases associated with apoptosis or diseases associated with abnormal gene expression. Viral disease means one caused by various viruses such as HIV, HCV and HBV.

In another embodiment of the present invention, there is provided a method of specifically cleaving a target nucleic acid using the chimeric molecule, complex or expression vector. Examples of the target nucleic acid include a gene derived from RNA virus, a protooncogene or a gene associated with apoptosis.

In another embodiment of the present invention, there is provided use of the chimeric molecule, complex or expression vector for analyzing the biological function of a target nucleic acid. Specifically, the present invention provides a method for analyzing a biological function of a target nucleic acid, including the method of specifically cleaving the target nucleic acid or specifically inhibiting a biological function of a target nucleic acid using the chimeric molecule, complex or expression vector, determining a sequence of the cleavage site of the nucleic acid and the neighborhoods as needed, and examining the influence of the cleavage or the inhibition on biological activity.

If any phenotype (e.g. the cure or suppression of canceration, or cell differentiation) is obtained by applying the method, a functional nucleic acid such as ribozyme acting effectively to do so is isolated, cloned and the sequence determined. Thus, on the basis of the obtained sequence, it is possible to determine the target gene of the functional nucleic acid. Using this method, it is possible to analyze the function of the target gene and find a novel gene.

As described above, any functional nucleic acid can be used in the present invention, but when ribozyme, antisense RNA or antisense DNA is used as a functional nucleic acid, the substrate-binding sites may be randomized. When hammerhead ribozyme is used as an example, its substrate-binding sites, stem I region and stem III region may be randomized. "Randomization" herein means the production of a pool into which all bases A, T(U), G and C corresponding to each base of substrate-binding sites of nucleic acid are introduced.

Specific terms used in the present specification are defined below.

"Sliding" means that a specific molecule moves on another molecule. In one embodiment of the present invention, by binding directly or indirectly a functional nucleic acid to a certain type of nucleic acid such as DNA or RNA (including mRNA) having binding affinity for a protein capable of sliding, the functional nucleic acid can move on nucleic acids via the protein capable of sliding.

"Functional region" refers to the region having specific biological functions *in vivo* or intracellularly, including enzyme function, catalytic function (e.g. RNA strand breaking activity), (biological) inhibitory function and (biological) promoting function. Examples of the region include ribozyme, DNA enzyme, antisense RNA, antisense DNA, aptamer, DNA enzyme (an enzyme expressing enzyme activity by incorporating metal), restriction enzyme etc.

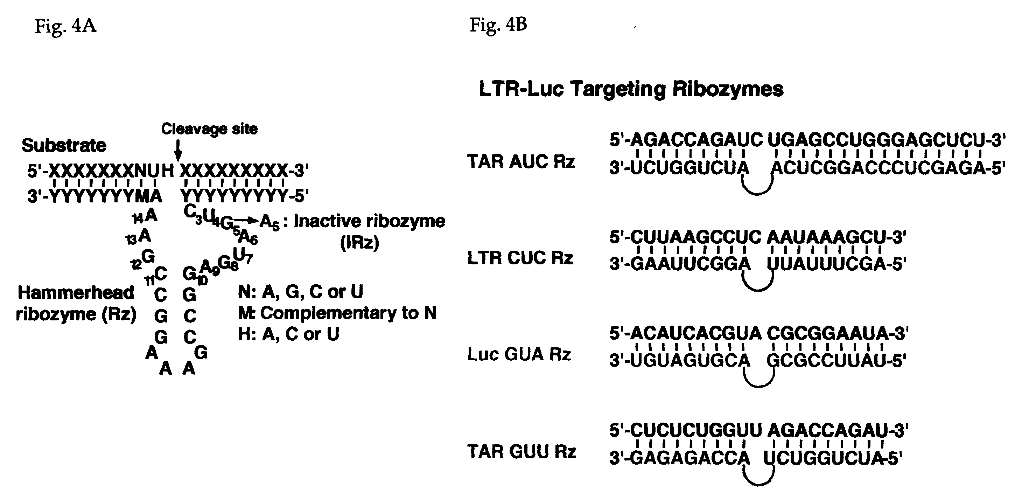

"Ribozyme" refers to RNA having catalytic function. "Catalytic function" refers to the action of specifically cleaving a specific site of RNA. A kind of ribozyme, hammerhead ribozyme binds to an RNA substrate (particularly mRNA), forming a complementary base pair, and cleaves the phosphoric diester bond at 3 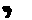 side of NUH sequence (N denotes A, G, C, U, and H denotes A, C, U. Any combination is available, but GUC is cleaved best.) (Fig. 4A). Ribozymes usable in the invention may be synthesized, isolated from nature, or may be commercially available products. Further, ribozymes usable in the invention contain a sequence complementary to any sequence of a target from any organism or virus. Ribozymes as shown in Fig. 4A are preferred, but the length of the sequence to which a target is attached is not limited to that indicated therein.

"Antisense RNA" or "antisense DNA" refers to a nucleic acid binding complementarily to a target RNA (particularly mRNA) or DNA, and inhibiting those functions. In the case where a target is mRNA, antisense RNA binds to mRNA, by which the translation to protein is inhibited.

"Aptamer" refers to an RNA molecule with high binding affinity for a specific protein. An aptamer acting specifically on a protein causing pathogenicity is considered to inhibit the function of the protein.

"RNA" or "DNA helicase" refers to an enzyme which bind to a single-stranded RNA or DNA to unwind their higher-order structure, and taking human as an example, it refers to a protein expressing in any cell.

"CTE (constitutive transport element)" refers to a cis-acting viral RNA which transports unspliced genomic RNA to cytoplam in order to express and package a virus protein. The terms "a nucleic acid having substantially equivalent functions to CTE" used in the present specification means a nucleic acid molecule having a function of transporting RNA to cytoplasm just as with CTE, and a nucleic acid molecule (RNA, DNA or the like) which is artificially created to have binding affinity for a sliding protein such as RNA helicase. Further, "the variant having substantially equivalent functions to CTE" used herein means a CTE variant having a function of transporting RNA to cytoplasm, and comprising a deletion, substitution or addition of one or more nucleotides in the CTE sequence. TAP (Tip-Associated Protein, Tip denotes tyrosine kinase-interacting protein herein.) or RNA helicase A can bind to CTE.

"Polymerase III (which is also called "pol III") promoter" means one suitable for expressing short RNA molecules such as ribozyme, and examples of the promoters include tRNA promoter, retrovirus LTR promoter, adenovirus VAl promoter etc.

"Terminator" refers to a gene positioning at mRNA transcription termination site.

"tRNA^{val} promoter (which is also called "tRNA^{val}") is a type of pol III promoter, and means a promoter associated with the transcription of short RNA molecules including tRNA. The tRNA^{val} promoter can have a sequence shown in SEQ ID NO: 8 or by formula (I) above or a DNA sequence encoding it, or variants thereof (e.g., nt. 1-91 of SEQ ID NO:10).

"Adapter" means one or more molecules intercalating between substances when the substances bind with each other. The binding may be covalent or noncovalent.

### Brief Description of the Drawings

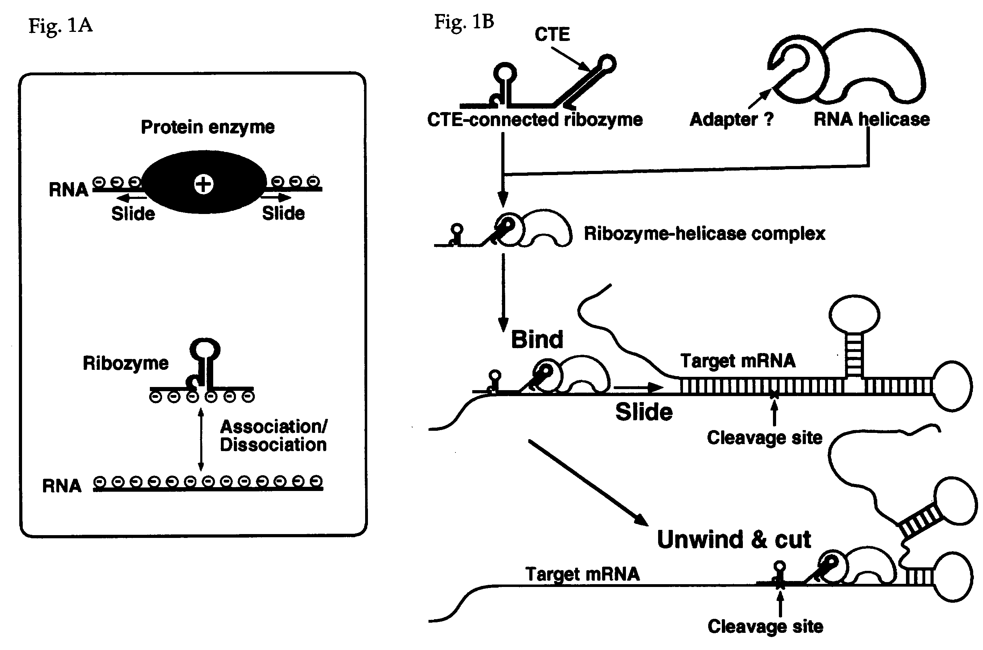

Figure 1: (***A***) Difference in slidability on RNA between a proteinaceous enzyme and a ribozyme, which is depicted shematically based on their charging. (***B***) Schematic diagram showing the addition of a CTE sequence and a possible sliding function to a hammerhead ribozyme and the cleavage of a hidden target site by a CTE-linked ribozyme.
Figure 2: (***A***) Predicted secondary structure of CTE as determined by MulFold. (***B***) Ribozyme-expression cassette controlled by a tRNA^{val} promoter.

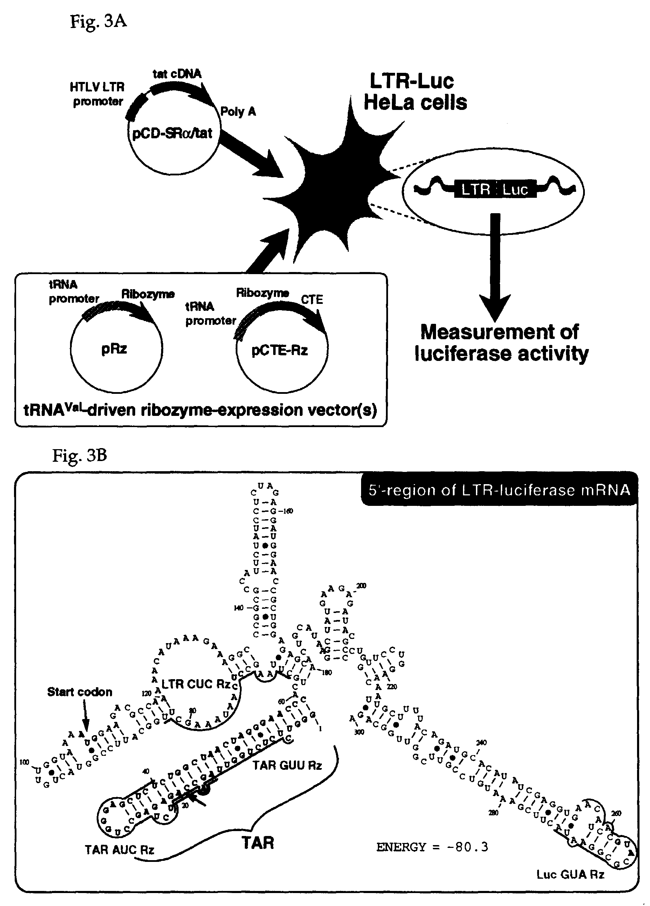

Figure 3: (***A***) Assay system for measurement of activities of tRNA^{Val}-ribozymes in LTR-Luc HeLa cell. (***B***) Predicted secondary structure of 5 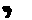 region of LTR-Luciferase mRNA targetted by ribozymes, as determined by MulFold.
Figure 4: (***A***) Secondary structure of a hammerhead ribozyme. (***B***) Ribozymes targetting LTR-fused luciferase mRNA.

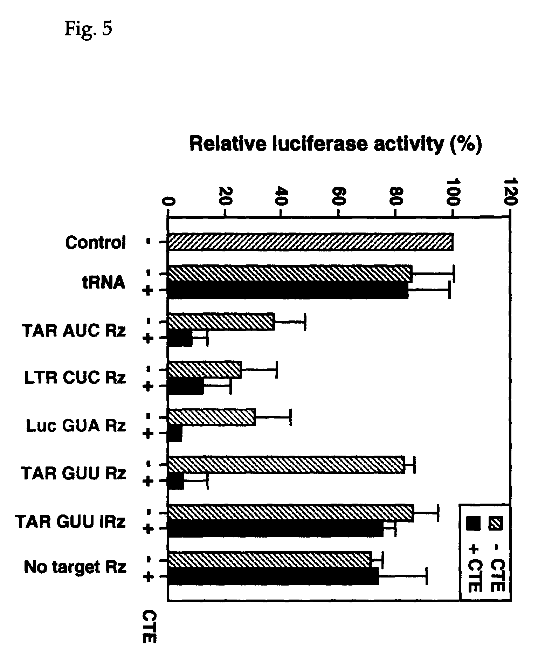

Figure 5: Inhibition of LTR-driven luciferase activity by tRNA^{Val} promoter-linked ribozymes.

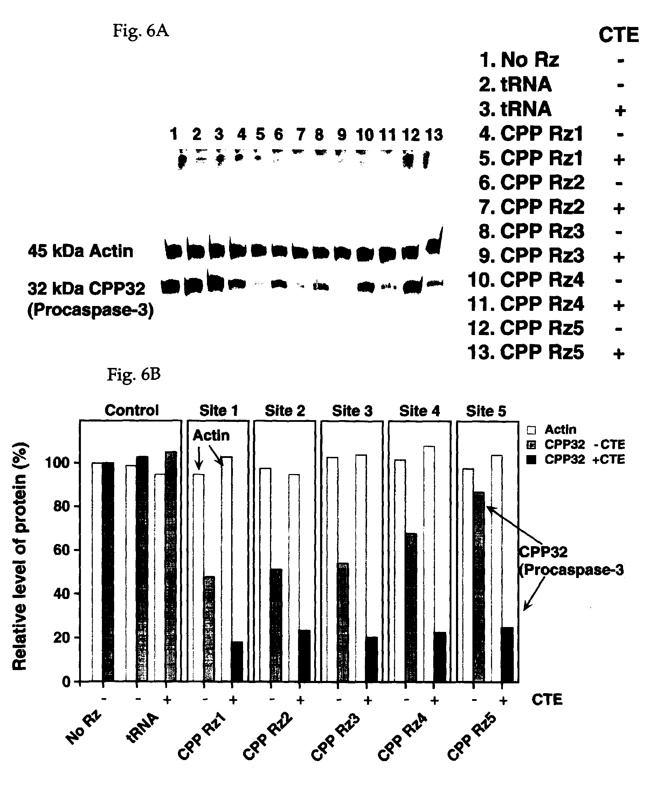

Figure 6: Inhibition of expression of CPP32 (Procaspase-3) by CTE-linked ribozymes and CTE-non-linkeded ribozymes. The results were obtained by analyzing the band intensity on Fluoro-imager (Molecular Dynamics) through Western blotting using FITC labeled α - IgG antibody as a secondary antibody (panel ***A***), and quantitating the intensity (panel ***B***),,

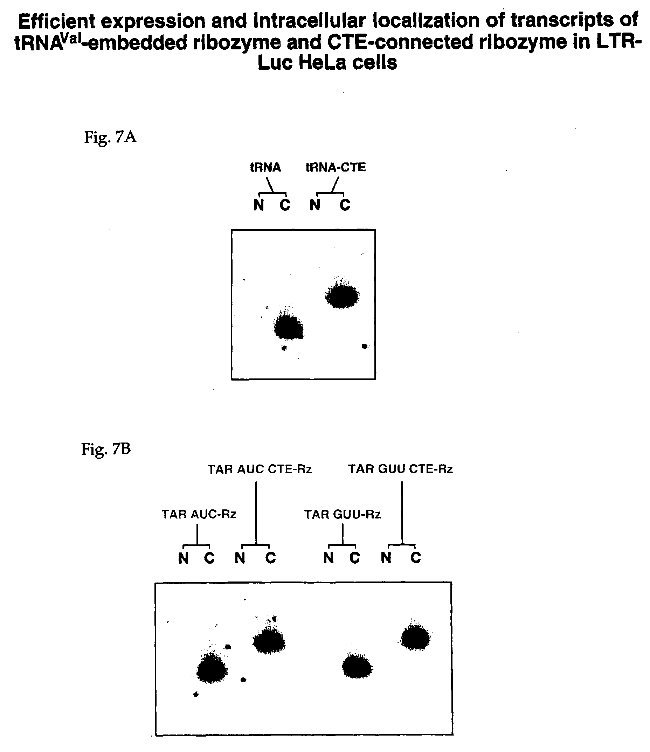

Figure 7: Effective expression and intracellular localization of tRNA-linked ribozymes and CTE-linked ribozymes in LTR-Luc HeLa cell. (***A***) Localization of tRNA-linked CTE. (***B***) Localization of tRNA-linked ribozymes and CTE-Rz. N indicates a nuclear fraction, and C a cytoplasmic fraction.

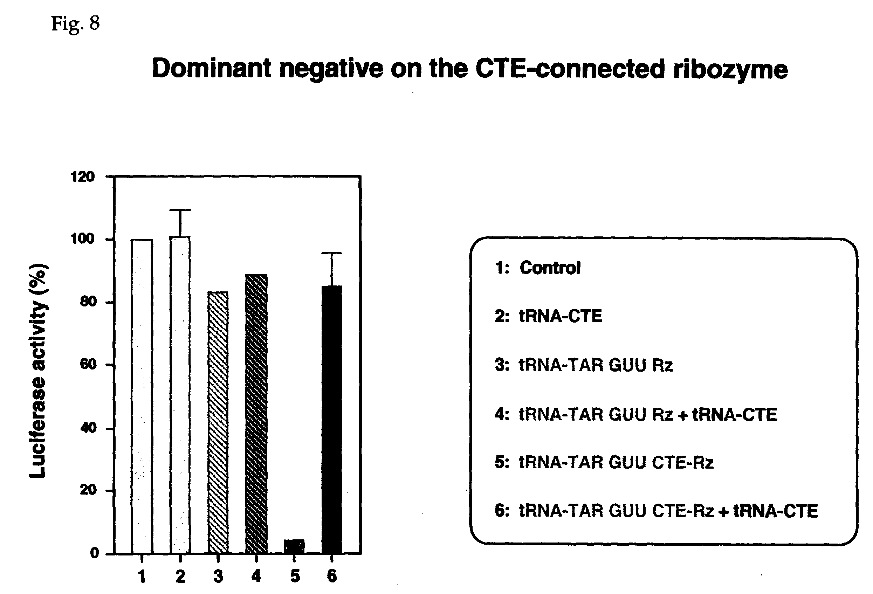

Figure 8: Dominant negative effect on CTE-linked ribozymes.

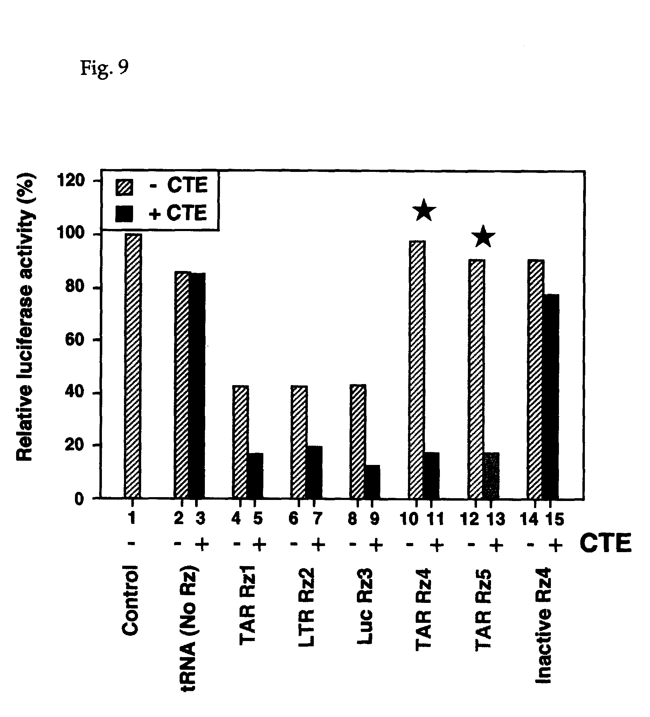

Figure 9: Suppression of LTR-driven luciferase activity by CTE-Rz. Red stars indicate results obtained with ribozymes targeted to relatively inaccessible sites of the TAR. LTR-Luc HeLa cells were transiently transfected with Tat alone (lane 1) or with Tat and the indicated tRNA-based ribozyme constructs (Koseki, S. et al. (1998) Journal of Controlled Release 53, 159-173). Luciferase activity as an indicator of ribozyme activity is reported as a percentage of the Tat only control. The presented values are the mean of at least three data points. Because the assays used transient transfections, there is statistical variability. The error bars are within 10% when data are taken on the same day and are 10%-25% when taken on a different day. However, in all cases, the CTE-ribozyme always had significantly greater activity than the conventional ribozyme.

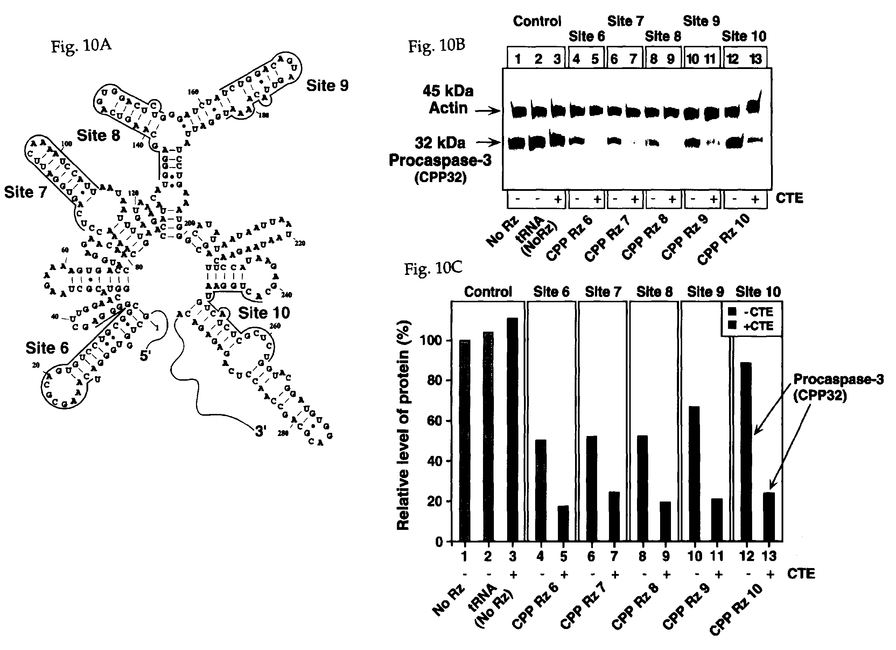

Figure 10: Inhibition of procaspase-3 (CPP32) gene expression by CTE-Rz. (***A***) The secondary structure predicted by MulFold (Jaeger, J. A. et al. (1989) Methods in Enzymology 183, 281-306) of the 5' region of procaspase-3 mRNA targeted by ribozymes. (***B***) Detection of procaspase-3 and actin proteins by Western blotting (Kuwabara, T. et al. (1998) Mol. Cell 2, 617-627). Mouse NIH3T3 cells were transfected with the indicated ribozyme constructs. (***C***) The results in B presented as a histogram. Fluorescein isothiocyanate-labeled (FITC-labeled) antibodies against rabbit IgG were used as the secondary antibody and the band intensities for actin and procaspase-3 were quantitated. Procaspase-3 protein levels were normalized to actin protein levels. The normalized level of protein recorded when cells were untransfected with the ribozyme-expressing vector was taken as 100% (lane 1).

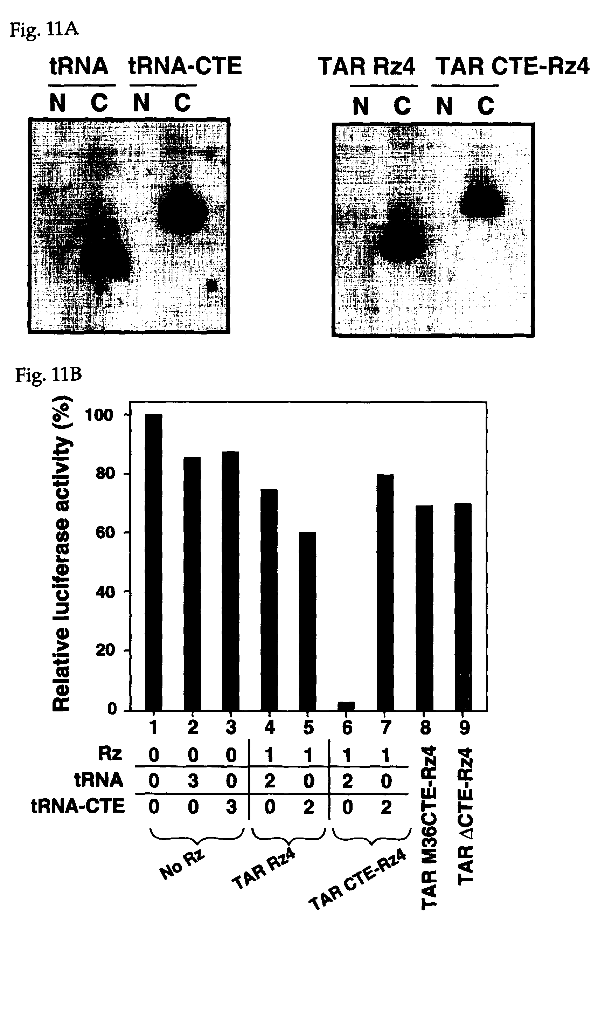

Figure 11: Effects of the CTE on ribozyme expression and mutant CTE on ribozyme activity. (***A***) Expression, stability and intracellular localization of ribozyme transcripts in HeLa cells. RNA prepared from HeLa cells transfected with the indicated expression constructs was fractionated. **N** and **C**, nuclear and cytoplasmic fractions, respectively. (***B***) Dominant negative effect of tRNA-CTE alone (compare lanes 6 and 7) and the effects of mutations in the CTE sequence on the activity of CTE-Rz (compare lanes 8 and 9). The amount in µg of plasmid that encoded each RNA used for transfection is indicated. The presented values are the mean of at least three data points. Because the assays used transient transfection, there is statistical variability. The error bars are within 10% when data are taken on the same day and are 10%-25% when taken on a different day.

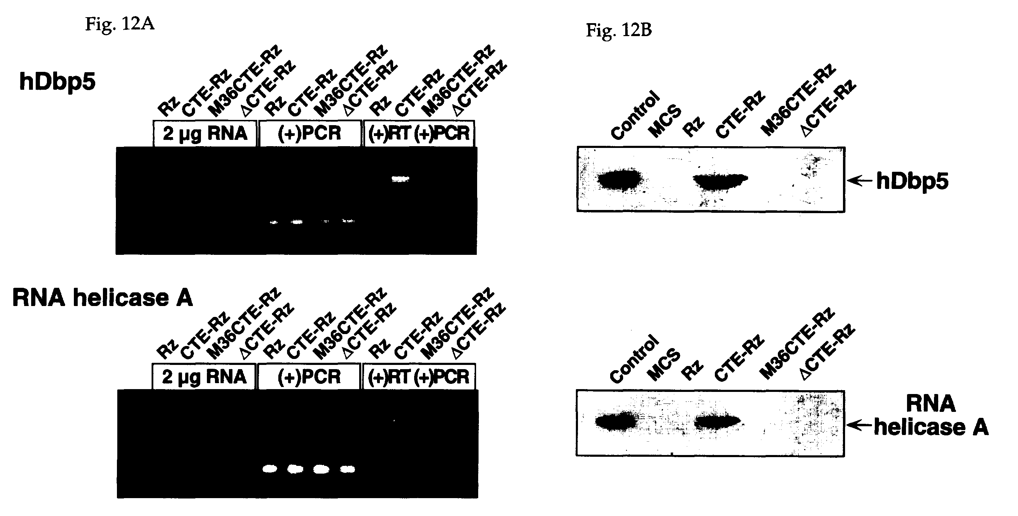

Figure 12: Interaction in cells between tRNA^{Val}-driven CTE-Rz and RNA helicases. (***A***) Co-immunoprecipitation of CTE-Rz RNA with RNA helicases hDbp5 and RNA helicase A (Li, J. et al. (1999) Proc. Natl. Acad. Sci. USA 96, 709-714). Either c-myc-hDbp5 (Schmitt, C. et al. (1999) EMBO J. 18, 4332-4347) or HA-RNA helicase A (Tang, H. et al. (1997) Science 276, 1412-1415 ; Li, J. et al. (1999) Proc. Natl. Acad. Sci. USA 96, 709-714) was transiently co-transfected into HeLa S3 cells with the indicated versions of the TAR Rz4 expression vectors. Immunoprecipitates obtained using the appropriate tag were subjected to RT-PCR ((+)RT (+)PCR). The CTE-Rz was only detected when wild-type CTE was present. As controls, the RNA was analyzed prior to RT-PCR (2µg RNA) and after being subjected to PCR without RT treatment ((-)RT (+)PCR). (***B***) RNA helicase hDbp5 and RNA helicase A interact with CTE-Rz. The indictaed versions of TAR Rz4 were *in vitro* synthesized using biotinylated UTP and then mixed with HeLa S3 cell extract from cells transfected with either c-myc-hDbp5 or HA-RNA helicase A (Li, J. et al. (1999) Proc. Natl. Acad. Sci. USA 96, 709-714). Streptavidin beads were used to precipitate the biotinylated RNAs and associated proteins. Western blotting using antibodies recognizing the appropriate tag revealed that only TAR CTE-Rz4 interacted with the helicases. *Control*, whole cell lysate from transfected cells.

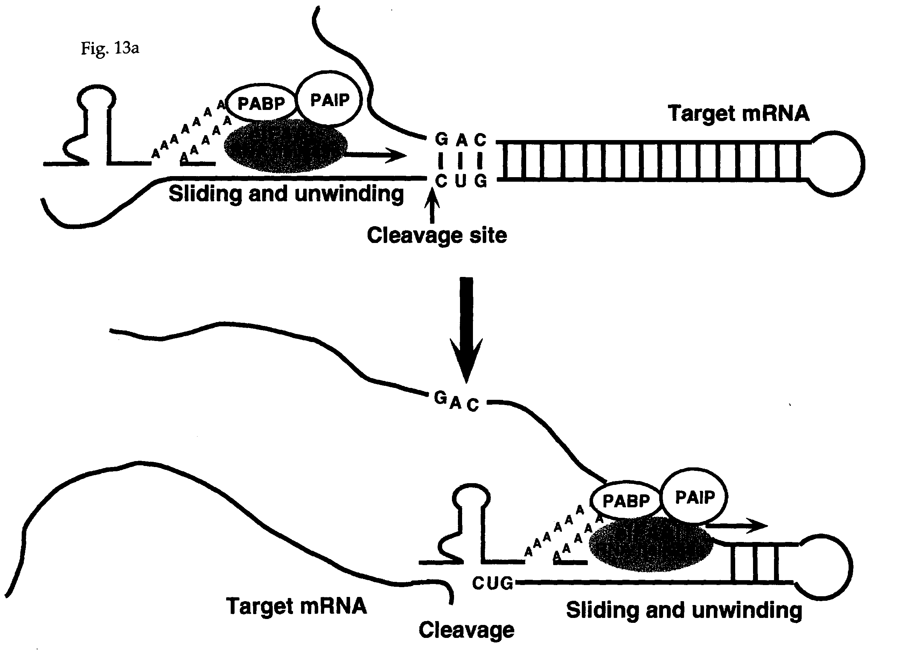

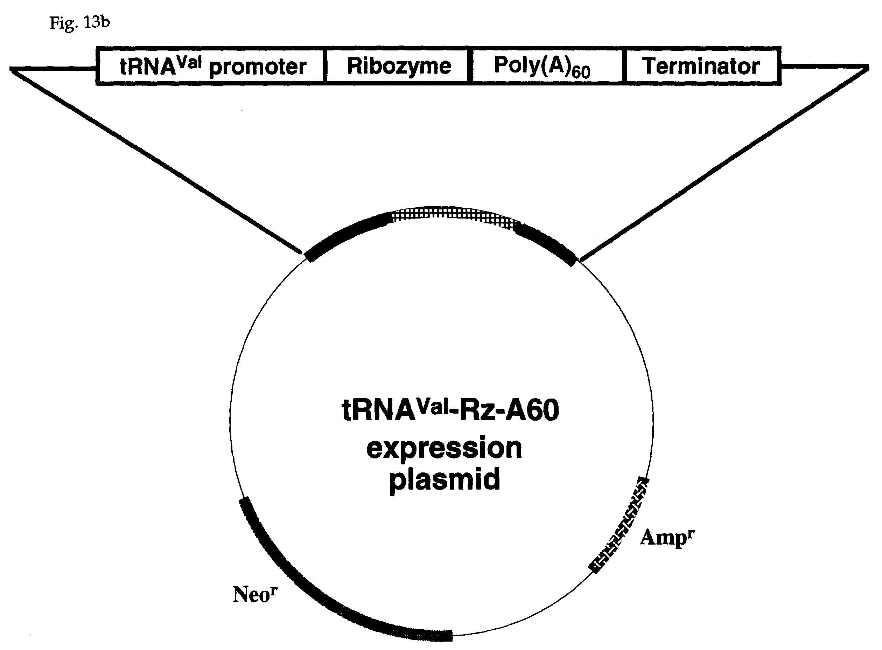

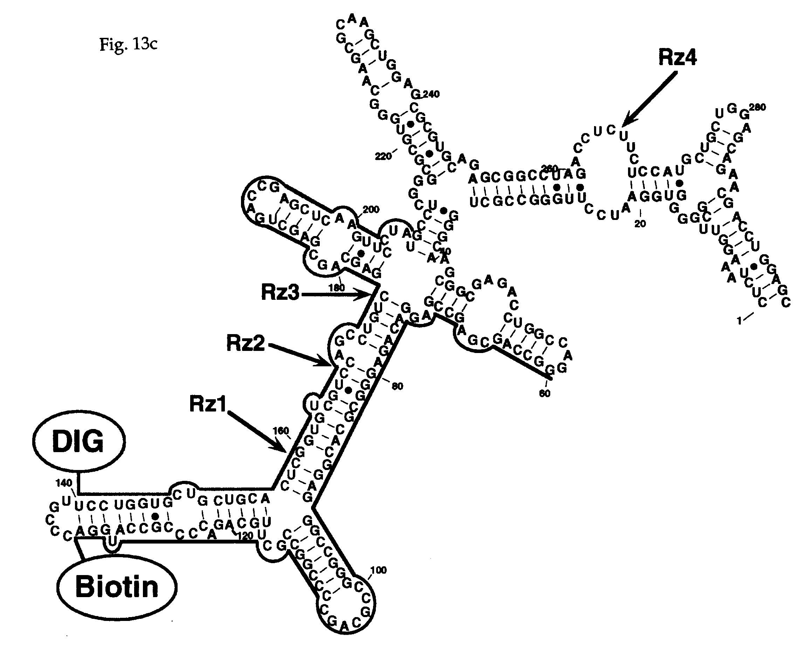

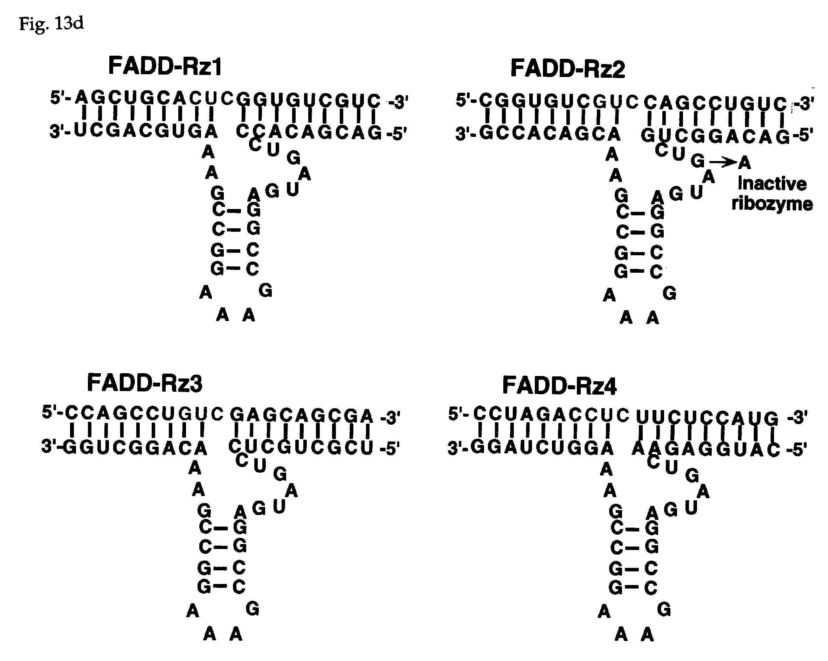

Figure 13: The concept of the poly(A)-linked hybrid ribozyme and the design of a ribozyme directed against the gene for human FADD. **a,** A schematic representation of the cleavage of a normally inaccessible target site by the hybrid ribozyme: addition of the poly(A) sequence allows recruitment of eIF4AI RNA helicase which couples the unwinding activity of the helicase to the cleavage activity of the hammerhead ribozyme. **b,** An expression cassette consisting of a poly(A)-linked ribozyme under the control of a tRNA^{Val} promoter. **c,** The secondary structure (as predicted by MulFold) of the 5' region of the mRNA for FADD that was the target of various ribozymes, as indicated. The parts of the mRNA for FADD used in later studies (Fig. 3) are indicated by green (nt. 60-134) and purple (nt. 140-206) lines. **d,** The various ribozymes used to target the mRNA. The sequences of the substrate-recognition arms of four ribozymes are shown. Cleavage occurs after the triplet shown in pink.

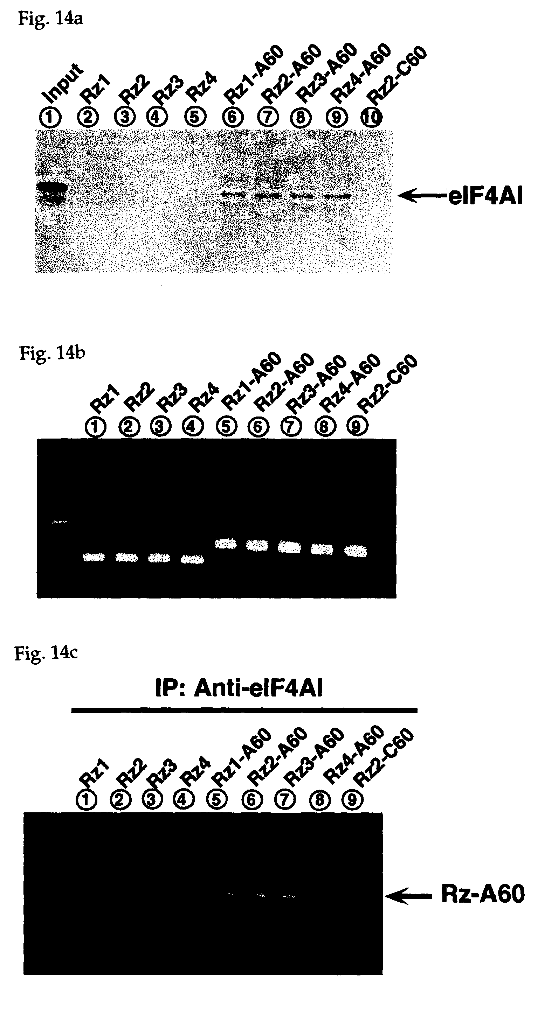

Figure 14: Interaction between the various types of tRNA^{Val}-driven poly(A)-linked ribozyme and the RNA helicase eIF4AI *in vitro* and *in vivo*. **a,** The RNA helicase eIF4AI interacts with each poly(A)-linked ribozyme. The indicated ribozymes were synthesized *in vitro* using biotinylated UTP and then mixed with extracts of HeLa-Fas cells. Streptavidin beads were used to precipitate the biotinylated RNAs and associated proteins. Western blotting, using a specific antibody against eIF4AI, indicated that only four poly(A)-linked ribozymes interacted with eIF4AI. See text for details. **b,** Expression of the various ribozymes in HeLa cells, as detected by RT-PCR. **c,** Immunoprecipitation of poly(A)-linked ribozymes with the RNA helicase eIF4AI. HeLa S3 cells were transiently transfected with the indicated ribozyme expression vectors. Immunoprecipitates were subjected to RT-PCR. Four poly(A)-linked ribozymes were co-immunoprecipitated with the RNA helicase eIF4AI.

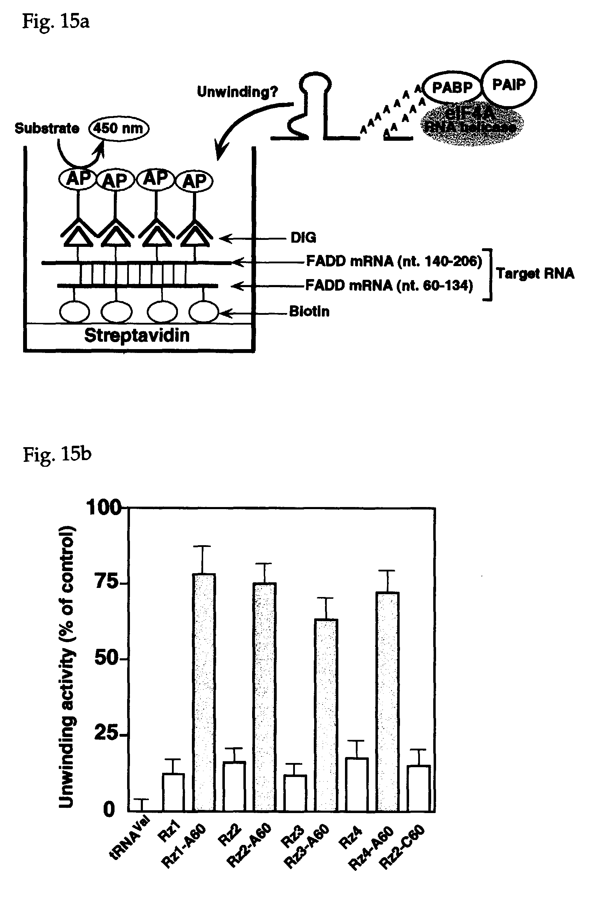

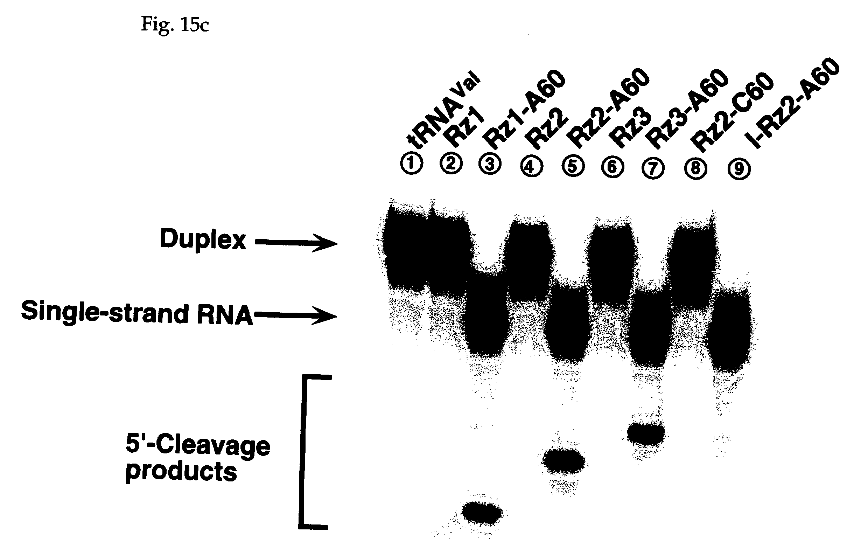

Figure 15: Detection by ELISA of helicase activity associated with poly(A)-linked ribozyme-protein complexes. **a,** Schematic representation of the ELISA that was performed as described in the text. The sequences of the substrate mRNAs for human FADD, that are indicated by green (nt. 60-134) and purple (nt. 140-206) lines, are given in Figure 13c. AP, Alkaline phosphatase; 450 nm, detection of the product of the reaction catalyzed by AP at 450 nm. **b,** Unwinding activity by hybrid ribozymes. All four poly(A)-linked ribozyme-protein complexes tested had helicase activity. Values are means with S.D. of results from 3 replicates in each case. **c,** Cleavage activity *in vitro* of poly(A)-linked or -non-linked ribozyme-protein complexes.

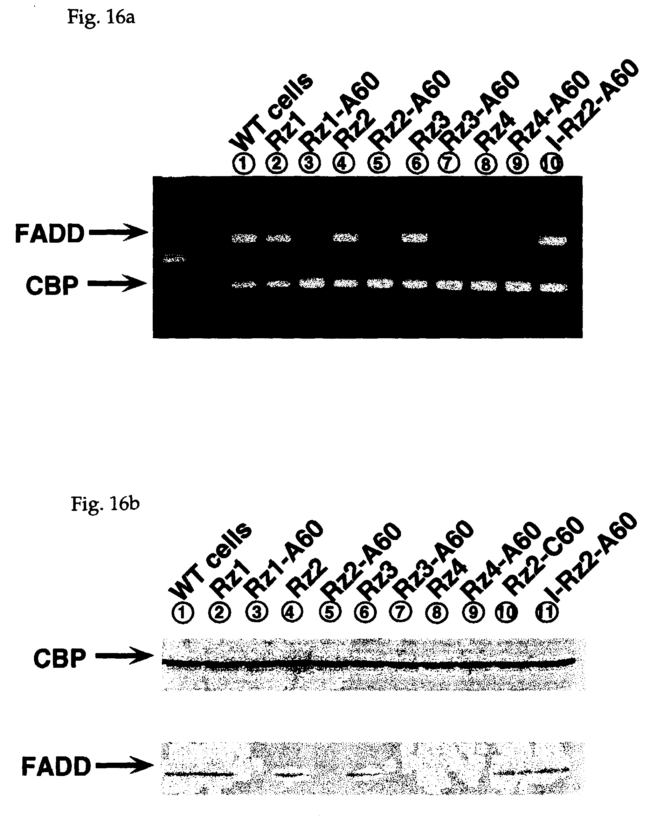

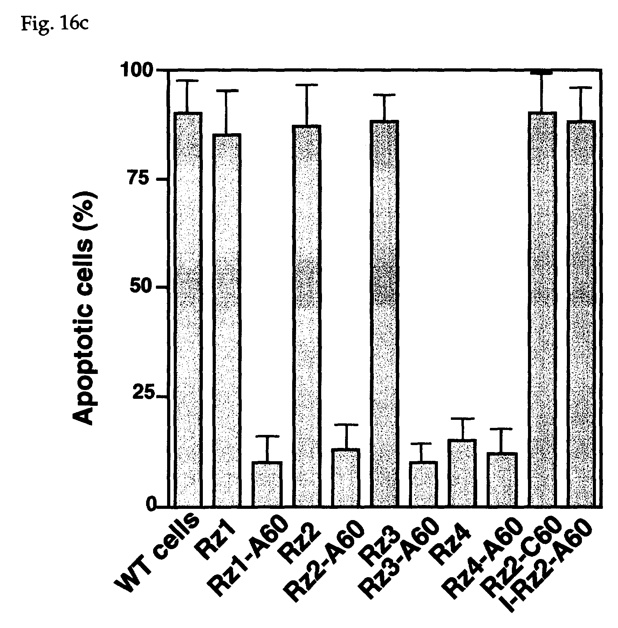

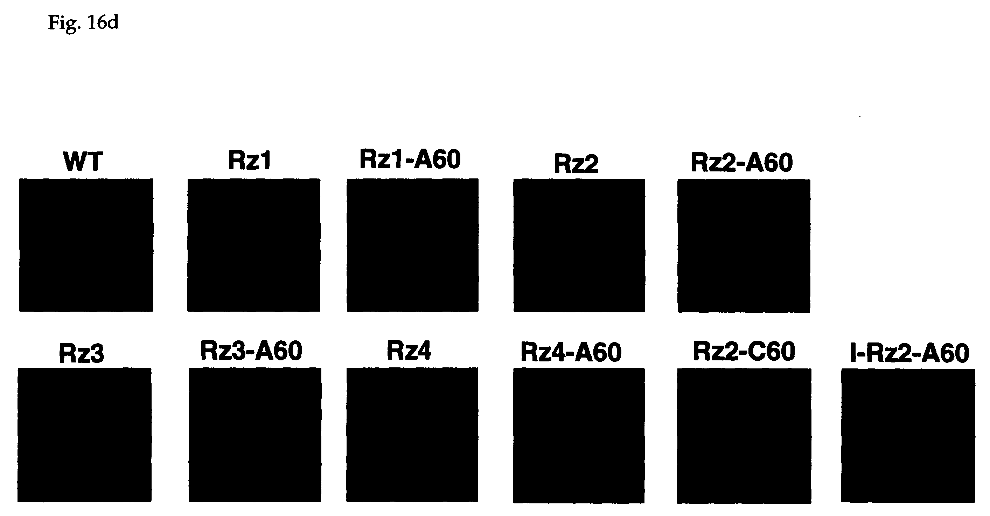

Figure 16: Inhibition of expression of the gene for human FADD by poly(A)-linked hybrid ribozymes. **a,** The levels of FADD mRNA in cells that expressed a poly(A)-linked or -non-linked ribozymes. **b,** The levels of FADD in cells that expressed a poly(A)-linked or -non-linked ribozyme. **c,** The extent of apoptosis (%) in cells that expressed a poly(A)-linked or -non-linked ribozyme. Values are means with S.D. of results from 3 replicates in each case. **d,** Detection of apoptotic bodies associated with the expression of a poly(A)-linked or -non-linked ribozyme.

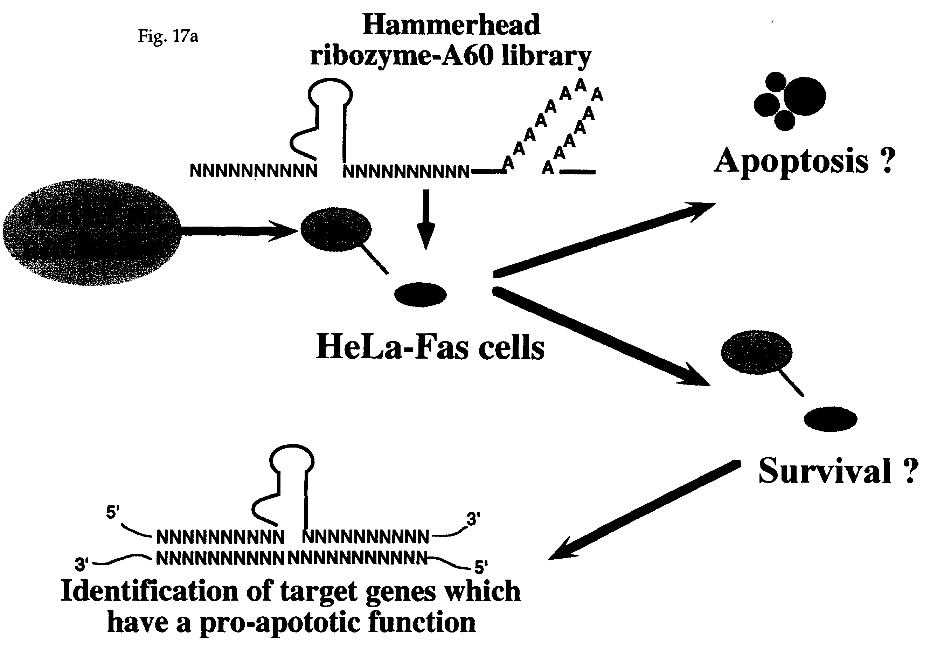

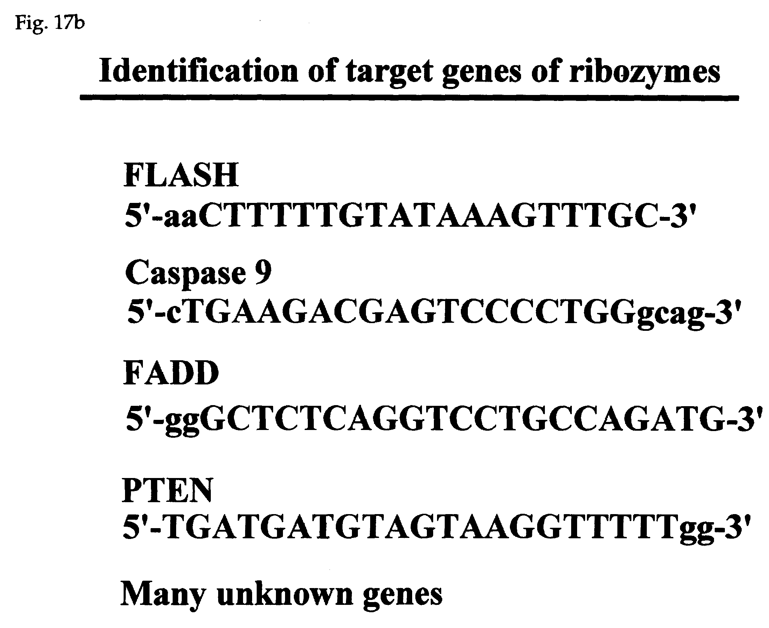

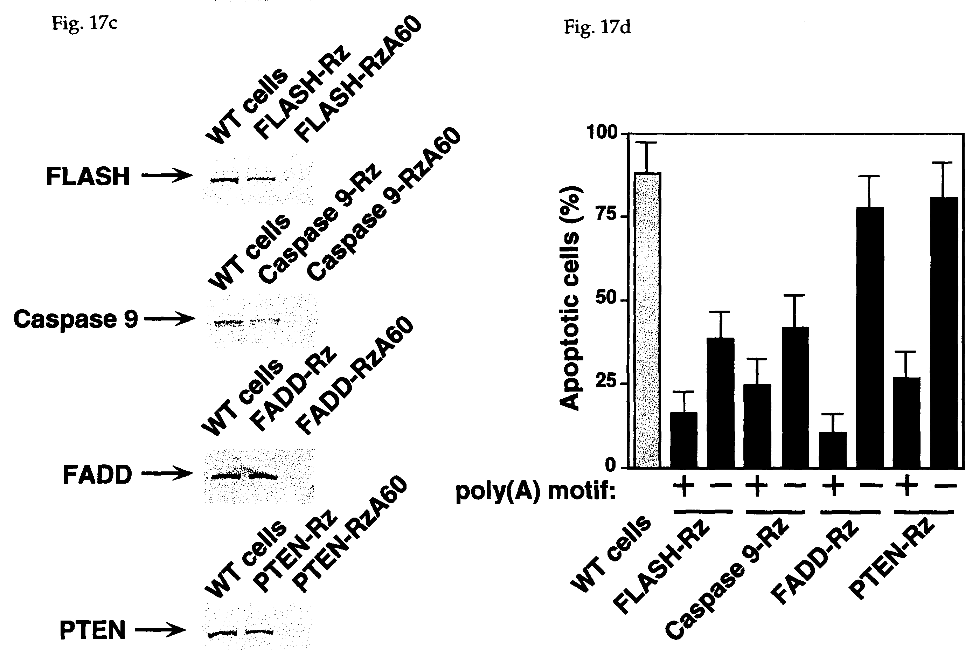

Figure 17: Gene discovery system for the Fas-induced apotosis by poly(A)-linked hybrid ribozyme libraries. **a,** Schematic diagram of the gene discovery system. HeLa-Fas cells that expressed the randomized Rz-A60 libraries were treated with the Fas specific antibodies. Sequences of the randomized arms of the ribozyme were obtained from the analysis of a genomic DNA that was isolated from survived clones. The important genes that are responsible in the apoptotic pathway can be identified from the gene data base. **b,** Identification of target genes by the gene discovery system. Capital letters indicate the target sequence that were complementary to the randomized arms of Rz-A60. **c,** The levels of expression of target genes in cells that expressed poly(A)-linked or -non-linked FLASH-, Caspase 9-, FADD- or PTEN-Rz. Human FLASH, Caspase 9, FADD and PTEN were detected by western blot analysis using specific antibodies against these factors. **d,** The extent of apoptosis (%) in cells that expressed a poly(A)-linked or -non-linked ribozymes directed against the gene for FLASH, Caspase 9, FADD or PTEN. Values are means with S.D. of results from 3 replicates in each case.

### Disclosure of the Invention

The present invention provides a chimeric molecule comprising a region with binding affinity for a molecule capable of sliding and any functional region; a chimeric molecule comprising a molecule capable of sliding and any functional region; and a chimeric molecule comprising a region with binding affinity for a molecule forming a complex with a molecule capable of sliding and any functional region.

Specifically, the present invention provides a chimeric molecule comprising a nucleic acid with binding affinity for a protein capable of sliding or a nucleic acid with binding affinity for a molecule forming a complex with the protein, and any functional nucleic acid.

As defined above, "functional nucleic acid" used in the present specification may be any nucleic acid (DNA or RNA) having a specific biological function *in vivo* or in a cell, and examples of the functional nucleic acid include, but are not limited to, ribozyme, DNA enzyme, antisense RNA, antisense DNA, aptamer, DNA enzyme (an enzyme expressing enzyme activity by incorporating metal) and so on. In Example described later, ribozyme, particularly hammerhead ribozyme is provided as a functional nucleic acid.

In addition, examples of the protein capable of sliding include, but are not limited to, helicase such as RNA helicase and DNA helicase and restriction enzymes such as EcoRV cleaving double-stranded RNA. If this protein binds exogenously or endogenously to functional nucleic acid, it is possible that the functional nucleic acid slides (or moves) on DNA or RNA. In particular, if helicase binds to a functional nucleic acid, the functional nucleic acid can slide on the target nucleic acid, unwinding its higher-order structure. In the present invention, both a chimeric molecule which comprises a nucleic acid sequence with binding affinity for a protein capable of sliding and a functional nucleic acid sequence bound thereto are produced, and, for example, the chimeric molecule is directly introduced into cell by applying methods including microinjection, by encapsulating the molecule within a cationic liposome, or by incorporating the molecule into a vector such as a viral vector and infecting the cell with it. Then, the chimeric molecule expresses in the host cell and acts on the target nucleic acid. Since the chimeric molecule of the present invention has binding affinity for a protein capable of sliding such as helicase or a molecule forming a complex with the protein, after being introduced into a cell, the chimeric molecule forms a complex with the protein or a molecule forming a complex with the protein therein, slides on a target nucleic acid, and accordingly the functional nucleic acid can act on the site of action existing on the target nucleic acid (see Figure 1). In particular, when the protein is helicase, since it slides on a target nucleic acid unwinding its higher-order structure, the functional nucleic acid effectively acts on the target.

Examples of "nucleic acid with binding affinity for a protein capable of sliding" or "a nucleic acid with binding affinity for a molecule forming a complex with a protein capable of sliding" include, but are not limited to, a nucleic acid with binding affinity for helicase or a molecule forming a complex with the helicase, preferably a nucleic acid with binding affinity for RNA helicase or a molecule (adapter) forming a complex with the RNA helicase (e.g. RNA sequence of approx. 170 nucleotides called CTE (constitutive transport element) binding to RNA helicase A (Figure 2A, SEQ ID NO:1)), poly(A) artificially produced RNA or DNA, etc. CTE is an RNA which monkey D retroviruses such as Mason-Pfizer monkey virus (MPLV) originally owns, and these viruses are considered to own an RNA motif, CTE, in order to carry out extranuclear transport for an unspliced RNA (H. Tang *et al*., (1997) *Science* 276:1412-1415; J. Li *et al*., (1999) *Proc*. *Natl. Acad. Sci.USA* 96:709-714; H. Tang *et al*.,(1999) *Mol. Cell Biol*. 19:3540-3550). In a preferable embodiment of the present invention, RNAs with binding affinity for a protein capable of sliding or a molecule forming a complex with the protein include the CTE, an RNA having substantially equivalent functions to the CTE, or a variant of CTE sequence shown in Figure 2A having substantially equivalent functions to the CTE. Herein, "an RNA having substantially equivalent functions to CTE" refers to a molecule other than CTE with binding affinity for a protein capable of sliding or a molecule forming a complex with the protein, for example, a molecule such as a helicase-binding aptamer binding to helicase artificially produced by SELEX method. "Variant" refers to an alteration (e.g., deletion, substitution or addition) of one or more nucleotides. This alteration can be carried out according to methods described in ordinary publications including J. Sambrook *et al, Molecular Cloning A Laboratory Mannual*, Cold Spring Harbor Laboratory Press (1989).

While not intending to be constrained by theory, it is thought that, when a host cell is transformed with a chimeric molecule comprising a CTE linked ribozyme or with an expression vector containing DNA encoding the chimeric molecule, as a result that RNA helicase binds to the CTE sequence which expressed in a cell, RNA helicase binding ribozyme is formed therein. And the helicase binds to a single-stranded part of target mRNA, unwinds the higher-order structure, sliding on RNA strand, while ribozyme detects the cleavage site existing inside of the target RNA strand and cleaves it.

In Examples of the present invention described later, the nucleic acid with binding affinity for a protein capable of sliding or a molecule forming a complex with the protein is CTE or poly(A), and the functional nucleic acid is a ribozyme. And, in this case, the protein capable of sliding is RNA helicase. However, the present invention is not limited to this example. Any nucleic acid with binding affinity for a protein capable of sliding or a molecule forming a complex with the protein can be used, and as the functional nucleic acid, not only ribozyme but also any functional nucleic acid such as antisense and aptamer can be used.

Generally, the chimeric molecule of the present invention can be obtained by, directly or indirectly binding a nucleic acid sequence with binding affinity for a protein capable of sliding or a molecule forming a complex with the protein, to a functional nucleic acid sequence, and for example it can be chemically synthesized by using a DNA/RNA synthesizer (e.g. model 394, Applied Biosystems). As a method of binding both nucleic acid sequences, the nucleic acid sequence with binding affinity for the protein capable of sliding may be upstream or downstream of the functional nucleic acid, preferably the nucleic acid sequence with binding affinity for the protein capable of sliding may bind downstream of the functional nucleic acid. Hereby, for example, the activity efficiency of the functional nucleic acid such as ribozyme is enhanced.

The present invention also provides an expression vector containing DNA encoding the chimeric molecule or this chimeric molecule. Examples of the vector constructing an expression system include plasmid vectors such as pUC19 (Takara Shuzo, Kyoto), pGREEN LANTERN (Life Tech Oriental, Tokyo) and pHaMDR (HUMAN GENE THERAPY 6:905-915 (July 1995)), and vectors for gene therapy such as adenovirus vector and retrovirus vector.

The above vector may comprise a promoter sequence upstream of the chimeric molecule. The promoter sequence is an element to control the expression of the chimeric molecule. Examples of the promoter include virus promoter (e.g. SV40 promoter), phage promoter (e.g. λ PL promoter), pol III promoter (e.g. human tRNA promoter such as tRNA^{val} promoter, adenovirus VAl promoter) etc. In the present invention, pol III promoter, particularly tRNA promoter is preferably used.

In addition, the vector of the present invention may comprise a terminator sequence downstream of the chimeric molecule. Any terminator sequence is used, provided that the sequence is one that terminates transcription. In Examples described later, UUUUU is used as a terminator sequence. The vector may optionally comprise a selectable marker gene or reporter gene such as an antibiotic resistant gene (e.g., Amp^{r}, Neo^{r}) or an auxotrophy complementing gene.

Furthermore, for the vector of the present invention, as a functional nucleic acid sequence, a functional RNA (e.g. ribozyme, antisense or aptamer) sequence is preferable; as the nucleic acid sequence with binding affinity for a protein capable of sliding or the molecule forming a complex with the protein, CTE or poly(A) sequence is preferable; and therefore a preferable chimeric molecule comprises a functional RNA sequence and CTE or poly(A) sequence.

The chimeric molecule of the present invention may be chemically synthesized by a DNA/RNA synthesizer, or may be obtained by collecting an RNA, that is, a chimeric molecule, after the RNA was synthesized using the above expression vector DNA as a template in the presence of DNA-dependent RNA polymerase enzyme. When the expression vector of the present invention is introduced into a cell, a chimeric molecule can express after it is inserted into a chromosome by homologous recombination. As a method of performing the homologous recombination, DNA encoding a chimeric molecule is inserted into a sequence homologous to a part of the host cell genome, then it is incorporated into a vector DNA. The insertion into a chromosome can be carried out not only with an adenovirus vector or retrovirus vector used in gene therapy, but with a plasmid vector as well.

Furthermore, the present invention comprises a complex of the chimeric molecule and a molecule capable of sliding. The chimeric molecule preferably comprises a ribozyme sequence and a CTE or poly(A) sequence. The protein capable of sliding is preferably helicase, more preferably RNA helicase. Either covalent bonding or noncovalent bonding can be used, unless the bonding impairs the function of each component. In the case of noncovalent bonding, the bonding can be done via an adapter specifically bonding to helicase. In the case of covalent bonding, it can be done via a linker as necessary.

Moreover, the present invention comprises a pharmaceutical composition containing the chimeric molecule, the complex, or the expression vector as an active ingredient. The pharmaceutical composition may include a pharmaceutically acceptable carrier (e.g. dilutent water such as physiological saline or buffer). The application of the pharmaceutical composition of the present invention depends on the function type of a functional region which is a component of the chimeric molecule. When the functional molecule is a ribozyme, antisense or aptamer, for example, it is effective at preventing or treating viral diseases such as AIDS virus (HIV), hepatitis C virus and hepatitis B virus, diseases associated with apoptosis such as Alzheimer's disease and Parkinson's disease, cancers, autoimmune diseases, inflammation, hereditary disease etc. Aptamer acts on the same target as ribozyme does. The functional nucleic acid of the present invention can impair the normal function of the causative agent, by cleaving or complementarily binding to the nucleic acid of the causative agent, or specifically binding to a pathogenic protein, so as to inhibit the function. The methods of introducing a chimeric molecule or a vector containing DNA encoding the molecule into cell include calcium phosphate method, electroporation, lipofection, microinjection, particle gun, the use of liposome (e.g. Mamoru Nakanishi *et al., Proteins, Nucleic Acids & Enzymes*, Vol. 44, No. 11, 1590-1596 (1999)), and the like. When a vector is used, it is introduced into a cell according to one of the above methods.
For example, part of cells may be removed from a diseased site to be subjected t*o in vitro* gene transfer and subsequently transplant back into tissue, or a vector may be directly introduced into the tissue of the diseased site. When a virus vector is used, the virus titer is usually more than approx. 10⁷ pfu/ml.

The present invention further provides a method of specifically cleaving a target nucleic acid, or specifically inhibiting or controlling a biological function of a target nucleic acid, using the chimeric molecule, the complex or the expression vector. In this method, the functional nucleic acid which is a component of the chimeric molecule is a ribozyme or antisense. For example, this method is also used for clarifying a biological function of a target nucleic acid. By randomizing the sequence of the target binding site of ribozyme (stems I and III), genes necessary for a certain biological function can be also clarified.

Specifically, a ribozyme having the above randomized target binding site is allowed to be capable of sliding and introduced into cell. For example, by introducing the ribozyme into a normal cell so as to cause canceration, or by introducing it into an abnormal cell so as to normalize the cell, it is possible to clarify genes associated with canceration. By analyzing the sequence through GenBank etc. as necessary, the entire sequences and functions can be determined. When the gene is unknown, the entire sequences may be determined by cloning the target gene, using the sequence of a target binding site as a clue. Even though the above randomized sequences are complementary to important genes, most of them can not interact with the target due to the higher-order structure of the target and end up not cleaving. Adding a sliding function solves the above problem and significantly enhances efficiency.

Now, ribozyme as a functional nucleic acid, CTE or poly(A) sequence as a nucleic acid with binding affinity for a protein capable of sliding or a molecule forming a complex with the protein are provided to describe the present invention.

The actual design of a highly functional ribozyme is described.

The first important point is the method of expressing a ribozyme in a cell. There are mainly two methods of introducing a ribozyme into a cell. The One is a method whereby a synthesized RNA molecule encapsulated within a cationic lipid membrane (liposome) etc. is directly introduced into a cell. For this method, various chemical modifications should be added to an RNA in order to give it resistance against ribonucleases existing in the cell. The other is a method whereby DNA encoding an RNA sequence is introduced into a cell using a plasmid, virus vector and so on and ribozyme is expressed by using the RNA transcription system existing in the cell. For this method, the effect of inhibiting the expression of a target gene is produced continuously, and there is no cytotoxic effect without having any modification that is added for the former method. The effect of inhibiting the expression of target gene by ribozyme depends largely on its transcription level, stability and posttranslational activity. Thus, it is important that the expression systems associated with these factors are well selected and an effective ribozyme expression vector is constructed.

As an expression system of ribozyme, the present inventors have selected RNA polymerase III system in which the expression level of ribozyme is larger by two or three orders of magnitude relative to RNA polymerase II system. In order to express ribozyme, it is necessary to add a promoter sequence that can be recognized by RNA polymerase III in front of a ribozyme (J. Ohkawa *et al*., (1993) *Proc. Natl. Acad. Sci. USA* 90:11302-11306 etc.). As a preferable promoter, the present inventors have found tRNA promoter, particularly tRNA^{val} promoter and have bound this to a ribozyme so that the promoter can act. As a preferable manner of binding, the ribozyme is positioned downstream of the promoter. Since the manner of binding has a great influence on the intracellular activity of ribozyme, how promoter sequences can be bound in front of ribozyme in order not to influence on the cleaving activity of ribozyme has been examined, and an effective ribozyme expression unit has been developed (S.Koseki et al., (1999) J. Viol. 73:1868-1877; T. Kuwabara et al., (1999) Proc. Natl. Acad. Sci. USA 96:1886-1891; T. Kuwabara et al., (1998) Mol. Cell 2:617-627). By these efforts, it has been determined that most of expressed ribozymes are transported in such a way that they are localized in cytoplasm. Hence, it becomes possible that ribozymes are localized in cytoplasm as well as the target mRNAs are.

### CTE linked ribozyme

It has been already described that an RNA sequence, CTE is introduced in order to bind a ribozyme to an RNA helicase, which has functions of binding to RNA, sliding and unwinding the higher-order structure of RNA, in a cell. The present inventors have inserted this CTE sequence between 3 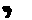 side of a ribozyme sequence and a terminator sequence via a short linker sequence (Figure 2B). The ribozyme expression vectors with or without the CTE are produced and their effects are compared in a model system wherein luciferase in HeLa cell is used.

The present inventors had produced a LTR-luciferase chimeric molecule (SEQ ID NO: 2) wherein luciferase gene was incorporated downstream of LTR region containing TAR (trans activating region) of HIV-1, and had established a transformed HeLa cell strain (LTR-Luc HeLa) having the luciferase gene incorporated into its genome (Figure 3A) (S. Koseki *et al.,* (1988) *J. Control. Release* 53:159-173). Regarding this LTR-luciferase chimeric molecule, the transcription is promoted by binding Tat protein(Trans-activating protein) to TAR region existing in LTR region, so that the expression of luciferase encoded downstream is also promoted (Figure 3B). That is to say, this chimeric molecule is a modeling of HIV transcriptional control system via TAR and Tat which are indispensable for the growth of HIV, and it is a simple assay system which can evaluate the effect of inhibiting the growth of virus without using the true virus. Regarding this cell, the expression of LTR-luciferase gene contained in a chromosome is induced by adding a Tat protein expression vector (*pCD-SR α/tat;* Y. Takebe *et al.,* (1988) *Mol. Cell Biol.* 8:466-472) exogenously. Then, if a ribozyme expression vector targeting LTR-luciferase gene or Tat gene is added at the same time, the effect of inhibiting the expression is measured from the luciferase activity. The assay system using such reporter genes can measure the effect of inhibitory agents such as ribozyme quantitatively, and seemed to be suitable for evaluating the ability of a highly functional ribozyme.

Regarding the cleavage site of ribozyme, hammerhead ribozyme, forming a complementary base pair with a substrate mRNA, binds to it and cleaves the phosphoric diester bond at 3 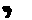 side of NUH sequence (N denotes A,G, C,U; H denotes A, C, U, any combination is available, but GUC is cleaved the most effectively) (Figure 4A). A large number of sites capable of cleaving of hammerhead ribozyme are contained in an LTR-luciferase chimeric molecule. As described above, a ribozyme has a poor ability to get into a rigid higher-order structure in a substrate RNA and cleaving a NUH sequence found therein, and the ribozyme prefers a NUH sequence existing in a loop or stem loop wherein it can form a complementary base pair with relative ease. Thus, regarding the region from the transcription initiation site to nucleotide 300, the structure of LTR-luciferase mRNA was deduced using a computer (Figure 3B). Out of this 5 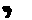 region, a ribozyme targeting NUH sequence existing in an easily accessible loop or stem loop and another ribozyme targeting for NUH sequence existing in a rigid stem which is unsuitable for the cleavage site of ribozyme were designed (Examples 1 and 2, Figure 4B etc.). Each target site is shown in Figure 3B (SEQ ID NO: 22 to 26). An RNA motif called TAR is contained in a transcript from LTR region. The TAR is an RNA motif having a stem loop structure and it has been well studied in view of the function and the higher-order structure. It is known that there exists a long stem region of more than 20 base pairs in the stem loop region and that it has an extremely rigid structure. Among the ribozymes designed herein, TAR GUU Rz (which is identical to TAR Rz4 in Fig. 9) and TAR Rz5 targets GUU and CUA sequences respectively existing in the rigid stem region of this TAR. With the previous conventional techniques, it is predicted that this type of ribozymes cannot bind to the cleavage site and the cleavage efficiency is significantly low. However, if an RNA helicase binds to a ribozyme through the ligation of CTE sequence to the ribozyme and an RNA helicase-bound ribozyme can act just as the present inventors have predicted, it is then expected that TAR GUU Rz and TAR Rz5 targeting the rigid stem region of TAR also exhibit a high cleavage activity. Hence, regarding 5 types of ribozymes, the present inventors have produced both a ribozyme with the addition of CTE (CTE-Rz) and another without the addition of CTE (Rz) as shown in Figure 2B, and have compared their cleavage activity in a cell.

### Inhibition of LTR-luciferase chimeric molecule activity by CTE linked ribozyme

The intracellular activity of CTE linked or CTE non-linked ribozymes to LTR-luciferase mRNA, as stated above, has been evaluated by measuring the luciferase activity in a cell wherein each ribozyme was introduced.

As an assay method, first, Tat protein expression vector (pCD-SR α/tat) and a CTE linked or CTE non-linked ribozyme expression vector (pCTE-Rz or pRz) were transfected into a LTR-Luc HeLa cell existing in a serum-free culture medium (OPTI-MEM-1) with lipofectin reagent (Figure 3A). After incubating these cells in a CO₂ incubator for 12 hours, they were transferred to a serum culture medium (DMEM, 10% FCS) and were further incubated for 24 hours. At the end of the incubation, the cells were lysed and the luciferase activity of the cell extract was measured.

The luciferase activity in the case of transfecting only Tat protein expression vector into cell was taken as 100%. The results are shown in figures 5 and 9. As clearly shown in the Figure, any ribozyme with CTE indicates a higher inhibitory effect than those without CTE do. In particular, although TAR GUU Rz and TAR Rz5 targeted TAR stem regions while the wild type could hardly inhibit the expression of LTR-luciferase gene. By adding CTE to a ribozyme, its activity dramatically increased thus showing a high inhibitory effect. In order to effectively cleave the RNA forming a rigid higher-order structure just as with TAR, not only the increase of RNA binding ability, but giving support to unwind the higher-order structure is also necessary. The high inhibitory effect of CTE-Rz targeting TAR would be easily explained, if it is postulated that such an effect is due to the addition of an unwinding ability of RNA helicase to a ribozyme as a supporter. Furthermore, as a control test, the assay was carried out in the same manner also for an inactivated ribozyme having a variant (TAR GUU IRz; Figure 4A) and a ribozyme which does not target LTR-luciferase mRNA (No target Rz), but a significant inhibitory effect was not obtained. From this result, it can be said that there is almost no side effects of the addition of CTE and that the action is specific for a ribozyme.

Apart from this experiment, the present inventors have produced CTE-Rz for several other types of genes and have evaluated their cleavage effect, and they have obtained the same results as stated above. Figures 6 and 10 show an example, and herein CTE is introduced into a ribozyme targeting for an important gene relatng to apoptosis, Procaspase-3 (which is also called CPP32 and is a precursor of Caspase-3 activating nuclease relevant to the fragmentation of chromatin caused by apoptosis). Also for this gene, 10 ribozymes were constructed followed by introducing each gene into NIH3T3 cell, and the expression level of Procaspase-3 protein was examined by western blotting. Figures 6A and 10B show the results obtained by reading by a fluoroimager, blots obtained from Western blotting wherein FITC labeled secondary antibody was used. These quantitate results are shown in figure 6B and 10C. In any case, the inhibitory effect on the gene expression increased significantly by the addition of CTE. In this case also, even though wild type CPP-Rz5 could hardly cleave, the addition of CTE brought a high inhibitory activity. On the other hand, CTE did not affect the expression level of Actin that is used as a control (Figures 6A and 10B). From this result, it is understood that there is no side effect caused by CTE linked and CTE non-linked ribozymes and that this effect is specific for Procaspase-3.

### Poly(A) linked ribozyme

Second example of the present invention is a new poly(A) linked ribozyme which was accomplished by combining the cleavage activity of the hammerhaed ribozyme with the sliding and unwinding activity (Jankowsky, E. *et al. Nature* **403**, 447-451. (2000)) of the endogenous RNA helicase eIF4AI.

It is clear that ribozymes are potentially useful tools for suppression of the expression of specific genes. Although many experimental trials have been successful (Kawasaki, H., *et al. Nature* **393**, 284-289 (1998) ; Kuwabara, T., *et al. Nature Biotechnol*. **16**, 961-965 (1998) ; Kuwabara, T., *et al. Mol. Cell* **2**, 617-627 (1998); Koseki, S., *et al. J. Virol.* **73**, 1868-1877 (1999) ; Tanabe, T. *et al. Nature* **406**, 473-474 (2000)), but it remains difficult to design an effective ribozyme-expression system that can be used *in vivo*. In many cases, the efficacy of ribozymes *in vivo* has proved to be lower than that expected from experiments *in vitro*, probably because it is difficult for a ribozyme to make contact with its target RNA and to locate its target site in cells. Many researchers have tried to develop ways to identify the best target sites for ribozymes and antisense oligonucleotides (Milner, N., Mir, K. U. & Southern E. M. *Nature Biotechnol.* **15**, 537-541 (1997) ; Patzel, V. & Sczakiel G. *Nature Biotechnol*. **16**, 64-68 (1998)), and previously the selection of an accessible target sequence was critical for success in the efficient suppression of a target gene.

In this study, the present inventors constructed a novel hybrid ribozyme, tRNA^{Val}-Rz-A60, and demonstrated that ribozymes of this type have strong cleavage activity and a substrate-unwinding activity. This construct appears to have two major advantages. First, these ribozymes show unwinding activity through their association with the RNA helicase eIF4AI. Second, since this helicase is utilized in general translation, these ribozymes can likely be co-localized with their target mRNAs with high efficiency.

Hybrid ribozymes of the invention suppressed the expression of the target mRNA more efficiently than did the parental ribozymes. Moreover, they were able to cleave the target mRNA at any chosen site, regardless of the putative secondary or tertiary structure in the vicinity of the target site. All our data indicate that the increased efficiency of the various hybrid ribozymes originated from the interaction of the poly(A)-tail with the RNA helicase(s) possibly through adaptor molecules, such as PABP and PAIP (Craig, A. W. *et al. Nature* **392**, 520-523 (1998) ; Gallie, D. R. *Gene* **216**, 1-11. (1998) ; De Gregorio, E. *et al. EMBO J.* **18**, 4865-4874. (1999)). All the poly(A)-containing ribozymes that the present inventors have constructed exhibited significant activity in cells (data not shown). In many cases, only poly (A)-containing ribozymes and not the parental hammerhead ribozymes suppressed the expression of the gene of interest. The present inventors have also obtained similar results using an another helicase-binding motif, the constitutive transport element (CTE), which associates with other types of RNA helicase, such as RNA helicases A and Dbp5. Useful RNA motifs are likely not limited to a poly(A)-tail and CTE, and it is likely that a large variety of hybrid ribozymes with unwinding function will be found to have general and broad applicability.

### Identification of functional genes using ribozyme

In addition to using these ribozymes to cleave a specific known target mRNAs, they can be used to identify genes associated with specific phenotypes in cells. This can be accomplished by creating ribozymes with randomized binding arms. The sequence of the human genome will soon become available and it will be extremely valuable to have methods for the rapid identification of important genes (Q.-X. Li *et al*. *Nucleic Acids Res.*,**28**, 2605-2612 (2000) ; P. J. Welch *et al*. Genomics, **66**, 274-283 (2000) ; M. Kruger *et al. Proc. Natl. Acad. Sci. USA* **97**, 8566-8571 (2000)). Since our hybrid ribozymes can attack any site, they can attack any mRNA. If libraries of hybrid ribozymes with randomized binding arms are introduced into cells, the genes associated with any changes in phenotypes can be readily identified by sequencing of the specific ribozyme clone (Fig. 17a-d).

The recruitment of endogenous helicases as a means of coupling unwinding with cleavage activity should also be applicable to other ribozymes and antisense molecules and the present inventors are exploring these possibilities. These studies demonstrate that a simple generally applicable modification of ribozymes, namely, the addition of a poly(A) sequence to the tRNA-driven ribozyme, makes it possible to target previously inaccessible sequences for cleavage. Furthermore, this ribozyme technology represents a powerful tool for the development of gene-inactivating reagents of both therapeutic and general importance and for the rapid identification of functional genes in the post-genome era.

The present invention will now be further explained by way of examples but the scope of this invention is not limited by these examples.

### Examples

### Example 1: CET linked ribozyme (I)

### Materials and Methods:

### Construction of CTE-linked ribozyme-expression vectors

A ribozyme-expression vector pUC-dt that can produce active ribosomes in manmalian cells was constructed according to the methods of S. Koseki et al. (J. Virol. 73:1868-1877 (1999)). The ribozymes were expressed under the control of a tRNA^{Val} promoter. A ribozyme sequence was attached to the 3 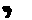 modified side of the tRNA^{Val} portion of the human gene to yield very active tRNA^{Val} promoter-linked ribozymes. The original plasmid, pUC-dt, had the promoter of a human gene encoding tRNA^{Val} and the cloning sites of Csp 45I and SalI for the insertion of a ribozyme.

The plasmid pUC-dt was double digested by restriction enzymes CspI and SalI. A ribozyme sequence with KpnI and EcoRV sites and the terminator sequence of UUUUU were cloned into this plasmid fragment. Subsequently, the CTE sequence was inserted into the KpnI and EcoRV sites (Fig. 2B). The inserted CTE sequence was SRV CTE-1 derived from monkey type D retrovirus. Fig. 2A shows its structure. A control plasmid that contains no CTE sequence but contains a ribozyme sequence and terminator sequence was also constructed.

As in the above procedures, four sets of ribozyme (TAR AUC Rz; LTR CUC Rz; Luc GUA Rz; TAR GUU Rz, which are shown in SEQ ID NOS: 4 to 7, respectively) with or without CTE sequence were cloned into the pUC-dt. For example, DNA sequences for the expression cassette sites of TAR GUU Rz and TAR GUU CTE-Rz are shown in SEQ ID NOS: 10 and 11, respectively.
(SEQ ID NO: 10):
   5 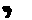 -accgttggtt tccgtagtgt agtggttatc acgttcgcct aacacgcgaa aggtccccgg ttcgaaaccg ggcactacaa aaaccaactt tatctggtct ctgatgaggc cgaaaggccg aaaccagaga gggtaccccg gatatctttt t-3 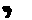
(SEQ ID NO:11):
   5 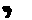 -accgttggtt tccgtagtgt agtggttatc acgttcgcct aacacgcgaa aggtccccgg ttcgaaaccg ggcactacaa aaaccaactt tatctggtct ctgatgaggc cgaaaggccg aaaccagaga gggtaccaga ccacctccct gcgagctaag ctggacagcc aatgacgggt aagagagtga cattgttcac taacctaaga caggagggcc gtcagagcta ctgcctaatc caaagacggg taaaagtgat aaaaatgtat cactccaacc taagacaggc gcagcttccg agggatttgg atatcttttt-3 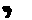

### Assay system for measurements of activities of tRNA^{Val}-ribozymes in LTR-Luc HeLa cells

LTR-Luc HeLa cells (Fig. 3A) that had been prepared by the methods of S. Koseki et al (S. Koseki et al., (1988) J.Control. Release 53:159-173) stably encoded chimeric molecules consisting of the LTR (long terminal repeat) of HIV-1 and luciferase gene. The LTR of HIV-1 contains regulatory elements that include a TAR (trans activating region for HIV) region (Fig. 3B). The HIV-1 regulatory protein, Tat, binds to TAR and the binding of Tat stimulates transcription substantially. Luciferase activity was measured by monitoring the effect of tRNA^{Val} promoter-linked ribozymes on the expression of the chimeric LTR-Luc gene (Fig. 3A).

Luciferase activities were measured basically according to the methods of S. Koseki et al., (S. Koseki et al., J. Control Release, 1998 supra). LTR-Luc HeLa cells were placed at 80% growth in a twelve-well plate and incubated at 37°C in a CO₂ incubator. Plated LTR-Luc HeLa cells were washed twice with phosphate-buffered saline (PBS) and placed in 300µL of serum-reduced medium (OPTI-MEM-1, Gibco BRL) before the (co)-transfection. In the assay shown in Fig. 3, 2µg of each ribozyme-expression plasmid and 150ng of Tat expressing plasmid (pCD-SRα/tat) were mixed with 3µL of Lipofectin reagent (Gibco-BRL, Rockville, MD) in 300µL of OPTI-MEM I medium, and incubated for 30 minutes at room temperature. After the incubation, the mixture was added gently to cells in OPTI-MEM-1 medium. After 12 hours, the medium was replaced by the growth medium (DMEM) with 10% FCS and cells were cultured for another 24 hours.

Luciferase activity was measured with a PicaGene Kit (Tokyo-inki, Tokyo, Japan) as described in the reference of Koseki et al., (S. Koseki et al., J.Control Release, 1998). Cultured LTR-Luc HeLa cells were washed twice with phosphate-buffered saline (PBS) and placed in 150µL of 1 x cell lysis buffer (Promega, Madison, WI). After the incubation for 30 minutes at room temperature, cells were scratched and the sediment was removed by the centrifugation. Appropriate aliquots were removed from each supernatant, mixed with 100µL of Luciferin (Tokyo-inki, Tokyo, Japan), and the luciferase activity was measured by a luminometer. In order to normalize the efficiency of transfection by reference to β-galactosidase activity, cells were co-transfected with 50µg of the pSV-β-galactosidase control vector (Promega, Madison, WI) and then the chemiluminescent signal due to β-galactosidase was determined with a luminescent β-galactosidase genetic reporter system (Clontech, Palo Alto, CA) as described in the user bulletin and reference of S. Koseki et al., (J. Control Release, 1998, *supra*).

For the assay shown in Fig. 8, LTR-Luc HeLa cells plated at 80% growth in a twelve-well plate were co-transfected with 1 µg of ribozyme-expression vector, together with 1 µg of the CTE-expression vector.

### Assay system for measurements of activities of tRNA^{Val} promoter-ribozymes which target the CPP32 (procaspase-3) mRNA

For the detection of the activities of CPP32-targetting ribozymes, the Western blotting analysis was performed to detect the level of CPP32 protein in NIH 3T3 cells (Fig. 6). NIH 3T3 cells which were transfected with each ribozyme-expression vector as described above were harvested. Fifty µg of the protein per lane were loaded on an 15% SDS PAGE and, after electrophoresis, bands of protein were transferred to a PVDF membrane (Amersham Co., Buckinghamshire, UK). The membrane was probed with a rabbit polyclonal αCPP32 antibody and a rabbit polyclonal actin antibody, followed by probing of the membrane with a FITC-conjugated IgG antibody as a second antibody. Then the bands were detected with Fluoro-Image Analyzer (Molecular Dynamics). The blocking and detection were basically performed as described previously (L. Dubrez et al., Blood, 1998, Blood 7:2415-2422).

### Northern blotting analysis

In order to determine the expression level of ribozymes and the intracellular localization of each ribozyme in LTR-Luc HeLa cells, Northern blotting analyses were carried out. Total RNA from LTR-Luc HeLa cells that had been transfected with each expression vector was separated into nuclear (N) and cytoplasmic (C) fractions.

Each ribozyme-expression vector indicated in Figure 7 was used to transfect LTR-Luc HeLa cells in combination with Lipofectin (Gibco-BRL, Rockville, MD). After culture for 36 hours at 37°C, total RNA was isolated with ISOGEN™ (Nippon Gene Co., Toyama, Japan). Cytopalsmic RNA and nuclear RNA were separated as described previously (Y. Huang and Carmichael, 1996, Mol. Cell. Biol., 16:1534-1542). Thirty µg of total RNA per lane was loaded on a 3.0% NuSieveTM (3:1) agarose gel (FMC Inc., Rockland, ME), and then bands of RNA were transferred to a Hybond-N™ nylon membrane (Amersham Co., Buckinghamshire, UK). The membrane was probed with synthetic oligonucleotides that were complementary to the sequences of respective ribozymes. The synthetic probe complementary to the sequence of CTE was used for the determination of the localization and the steady-state level of tRNA^{Val} driven CTE RNA. All probes were labeled with ³²P by T4 polynucleotide kinase (Takara Shuzo Co., Kyoto). The sequences of the probes are as follows.
A probe for tRNA (SEQ ID NO:12)
   aagatatccg gggtaccaaa gttggttttt gtagtgcccg
A probe for tRNA-CTE (SEQ ID NO:13)
   aagatatcca aatccctcgg aagctgcgcc tgtcttaggt
A probe for TAR AUC-Rz and TAR AUC CTE-Rz (SEQ ID NO:14)
   agaccagatt tcggcctttc ggcctcatca gtgagcctgg
A probe for TAR GUU-Rz and TAR GUU CTE-Rz (SEQ ID NO:15)
   ctctctggtt tcggcctttc ggcctcatca gagaccagat

Examples pertaining to the present invention will now be given with reference to figures.

Figure 1 is a schematic representation for addition of the CTE sequence and a possible sliding function to a hammerhead ribozyme; the cleavage of a hidden target site by a CTE-linked ribozyme.

In general, DNA interacting regions of restriction enzymes, such as those of EcoRV, are positively charged, which enables them to search for their target site by sliding along a chain of polynucleotides (Fig. 1A, linear diffusion, sliding mechanism; Jeltsch et al., 1996, EMBO J., 15: 5104-5111). In this mechanism, kinetically unfavorable repetitive association/dissociation events can be avoided during the search for the target site. By contrast, RNA-cleaving ribozymes are negatively charged as well as their RNA substrates. Therefore, ribozymes cannot slide along the RNA chain and thus they must search for their target sites by kinetically unfavorable repetitive association/dissociation events (Fig. 1A). As a result, the longer the RNA chain that contains the target site of a ribozyme, the lower the efficiency of cleavage by the ribozyme. Moreover, some target sites are not accessible to the ribozyme not only *in vitro* but also *in vivo* because some of them are hidden within stable stem structures.

In order to improve this situation, the present inventors have planned to install a slinding function to the ribozyme. RNA helicase which is taking place in the translation mechanism is a natural enzyme that can slide along mRNA and unwind the tertiary structure of the mRNA (C. -G. Lee et al., (1993) J. Biol. Chem. 268:13472 - 13478). Therefore, the inventors have tried to conjugate the ribozyme with RNA helicase by connecting both components with CTE through direct or indirect interactions (Fig. 1B). The RNA helicase can guide the ribozyme to its target site during unwinding of the structured mRNA because of its nonspecific RNA binding and sliding activities. Although the direct interaction of the RNA helicase with CTE was previously suggested, an indirect association through adapter molecules and involvement of another RNA helicase should also provide a sliding function to the ribozyme and enhance the activity of the ribozyme *in vivo.*

Figure 2A shows a predicted secondary structure of CTE based on MulFold. Figure 2B is a schematic representation of a ribozyme-expression cassett controlled by a tRNA^{Val} promoter.

Successful inactivation by ribozymes of a specific gene *in vivo* depends not only on the selection of the target site but also on the design of the expression vector. The latter determines both the level of expression and the half-life of the expressed ribozyme. While pol II promoters (for example, insulin promoters specifically functioning in spleen cells) might allow tissue-specific or regulatable expression, pol III transcripts might be expressed at significantly higher in levels. High-level expression under control of the pol III promoter would be advantageous because it is generally believed that the association of a ribozyme with its target mRNA is the rate limiting step of the ribozyme-mediated reaction in cells. Therefore, the inventors chose to express the ribozymes under the control of the promoter of a human gene for tRNA^{Val} promoter, which has previously been used successfully in the suppression of target genes by ribozymes (M. Yu et al., 1999, Proc. Natl. Acad. Sci. USA, 92:699-703; Bertrans et al., 1997, RNA 3:75-88).

The specific design of the tRNA^{Val} promoter-linked ribozyme enzymes was based on the inventors' previous success in attaching a ribozyme sequence to the 3 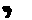 modified side of the tRNA^{Val} promoter portion of the human gene to yield very active ribozymes (H. Kawasaki et al., (1998) Nature 393:284-289; T. Kuwabara et al., (1998) Nature Biotechnology 961-965; T. Kuwabara et al., (1998) Mol. Cell 2:617-627; S. Koseki et al., (1999) Proc. Natl. Acad. Sci. USA 96:1886-1891). In all cases, the 3 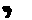 side of the tRNA^{Val} portion of the human gene were modified, so that (i) the transcript would not be processed by RNase P; (ii) the structure of the transcript would be sufficiently similar to the tRNA so as to allow recognition by an export receptor for export to the cytoplasm and to ensure co-localization with its target; and (iii) the substarte-recognition arms would be more accessible upon disruption of the intramolecular stem.

For the construction of CTE-linked ribozyme (CTE-Rz), CTE sequence, shown in Figure 2A, was inserted between the KpnI and EcoRV sites downstream of the ribozyme sequence of tRNA-linked ribozyme.

Figure 3A shows an assay system for measurements of activities of tRNA^{Val}-ribozymes in LTR-Luc HeLa cells. Figure 3B shows a predicted secondary structure based on MulFold of the 5 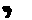 -region of LTR-Luciferase mRNA targeted by the ribozymes (SEQ ID NO:2).

The present inventors evaluated the intracellular activities of the tRNA^{Val}promoter-linked ribozymes and tRNA^{Val} promoter-linked and CTE-linked ribozymes, using LTR-Luc HeLa cells that stably encoded a chimeric gene which consisted of the long terminal repeat (LTR) of HIV-1 and a gene for luciferase (Koseki et al., (1998) J. Control Release 53:159-173). The LTR of HIV-1 contains regulatory elements that include a TAR region. The HIV-1 regulatory protein, Tat, binds to TAR and the binding of Tat stimulates transcription substantially. Measurements of luciferase activity allowed the inventors to monitor the effects of tRNA^{Val} promoter-linked ribozymes on the expression of the chimeric LTR-Luc gene. LTR-Luc HeLa cells were co-transfected with tRNA^{Val}-driven ribozyme-expression vector, together with a Tat expression-vector (pCD-SRα/tat) (Y.Takebe et al., (1988) Mol. Cell Biol. 8:466-472). After transient expression of both Tat and tRNA^{Val}-linked ribozymes, the inventors estimated the intracellular activity of each tRNA^{Val}-ribozyme by measuring the luciferase activity (Fig. 3A).

Figure 3B shows the predicted secondary structure of 5 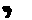 partial sequence of 300 nucleotides of the LTR-driven luciferase mRNA. Successful inactivation by ribozymes of a specific gene *in vivo* also depends on the selection of the target site. Some target sites are not accessible to the ribozyme not only *in vitro* but also *in vivo* because some of them are hidden within stable stem structures. In order to compare the efficiencies of gene inactivation by ribozymes that target relatively unaccessible sites, the inventors designed four sets of CTE-linked and non-linked ribozymes. Three ribozymes of TAR AUC Rz, LTR CUC Rz and Luc GUA Rz (labeled orange, blue and green, respectively) are designed to target relatively accessible sites that are located in loop regions of LTR-luciferase chimeric mRNA, as indicated by underlines with corresponding color in Figure 3B. By contrast, the remaining TAR GUU Rz (labeled red) is designed to target the inaccessible site that is located in the stable stem structure within the TAR region, as indicated by underline with red. It is well known that the TAR region has a stable stem-loop structure and so the inventors expected that this ribozyme could not show enough inhibitory effect in usual. Essential triplets for cleavage by hammerhead ribozymes are indicated by red letters.

Figure 4A shows a secondary structure of a hammerhead ribozyme. Figure 4B shows a series of ribozymes targeting the HIV-1 LTR-driven luciferase mRNA.

Hammerhead ribozymes are among the smallest catalytic RNAs. They are named "hammerheads" because their two-dimensional structure resembles that of a hammerhead. The sequence motif that is responsible for the self-cleavage reaction (cis action) was first recognized in the satellite RNAs of certain viruses. However, hammerhead ribozymes have been engineered in the laboratory to be able to act "in trans," in order to apply for the treatment. The trans-acting hammerhead ribozymes consist of an antisense section and a catalytic core with a flanking stem-loop section (Fig. 4A). These ribozymes can cleave oligonucleotides at specific sites NUH (wherein N indicates A, G, C or U; H indicates A, C or U), and can cleave a GUC triplet most efficiently.

The sequences of substrate-recognition arms of four ribozymes are shown in Figure 4B and labeled with corresponding color.

Figure 5 shows the suppression of LTR-driven luciferase activity by the tRNA^{Val}-linked ribozymes.

The luciferase activity recorded when only the Tat-expressing vector (pCD-SRα/tat) was used was taken as 100 % (lane 1). Ribozyme(s)- and Tat-expression vectors were used at a molar ratio of 10:1 for co-transfection of LTR-Luc HeLa cells. 36 hours after transfection, luciferase activity in each cell was analyzed. The results shown in Fig. 5 are the mean results from two sets of experiments. As can be seen from the figure, both tRNA and CTE linked tRNA had little inhibitory effect on the expression of the LTR-luciferase gene (lanes 2 and 3), indicating that there is almost no side effect derived from CTE and the enzyme-expression system using human gene for tRNA^{Val} promoter. By contrast, as anticipated, three types of ribozyme (TAR AUC Rz, LTR CUC Rz, Luc GUA Rz, lanes 4, 6, and 8), which are designed to target relatively accessible sites, had significant inhibitory effects on the expression of the luciferase gene. By contrast, TAR GUU Rz which was designed to target the inaccessible site in the stem-structured TAR region had almost no inhibitory effect (lanes 10).

To our surprised, when the CTE sequence was attached to the 3 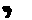 side of any ribozyme sequence (when the CTE-Rz's are used), in all cases, the efficiencies of ribozymes were remarkably improved to the same level (lanes 5, 7, 9, and 11). The efficiency of ribozymes targetted to both accessible and inaccessible sites increased by the same significant extent. In particular, TAR GUU CTE-Rz (lane 11) had a significant inhibitory effect (as well as other CTE-Rz) despite the fact that its parental TAR GUU Rz (lane 10) had almost no inhibitory effect. In this case, unwinding of the stem-structured TAR region is essential to exhibit such effective inhibition of luciferase activity. Therefore, this improvement in the inhibitory effect strongly supports the involvement of a certain effector molecule which can promote ribozyme-binding to its substrate and also unwind the highly structured mRNA. One strong candidate appears to be RNA helicase A, which demonstrates enzymatic activities of RNA binding, sliding and unwinding. As control, the inventors used an inactivated version of TAR GUU Rz (TAR GUU IRz) that had a single mutation at the catalytically important conserved nucleotide, G⁵ → A⁵, and a ribozyme with a target site which is not located within the LTR-Luc mRNA (No target Rz). Regardless of the presence or absence of the CTE sequence, these ribozymes had little inhibitory effect on the expression of the LTR-luciferase gene (lanes 12, 13, 14 and 15), indicating that there is almost no side effect derived from CTE or the enzyme-expression system using the human gene for tRNA^{Val}.

Figure 6 shows inhibition of expression of a gene fro CPP32 (Procaspase-3) by CTE-linked and non-linked ribozymes.

Different from the ribozymes targeted to the LTR-Luc mRNA, in order to investigate the generality of the effect of CTE-linked ribozyme, the inventors designed CTE-linked ribozymes that targeted to several other genes. In most cases, the inventors observed similar high efficacy of CTE-linked ribozymes. One example of such an experiment is shown here. The inventors have tried inhibiting the expression of a gene for mouse CPP32 (Procaspase-3) which is a well known apoptosis related gene. Five ribozymes (CPP Rz1 (SEQ ID NO:49), CPP Rz2 (SEQ ID NO:50), CPP Rz3 (SEQ ID NO:51), CPP Rz4 (SEQ ID NO:52), CPP Rz5 (SEQ ID NO:53)) for different sites in CPP32 mRNA were designed. In particular CPP Rz 5 was designed to target a relatively inaccessible site in the stem-structured CPP32 mRNA. Mouse -derived NIH3T3 cells were transfected with these ribozyme expression plasmids and 36 hours after transfection, protein content within each cell was determined by Western blotting analysis using α-CPP32 antibody. In addition, α-Actin antibody was used as a control. FITC-labeled α-IgG antibody was used as the second antibody and the intensity of the bands were analyzed (Fig. 6A) and quantitated (Fg. 6B) by a Fluoro-imager (Molecular Dynamics).

As shown in both Figures 6A and 6B, by connecting CTE to each ribozyme, the inhibitory effects were significantly enhanced, similar to the experiment shown in Figure 5. Even in this case, CTE-linked CPP Rz 5 showed enough inhibitory effects despite the fact that its parental ribozyme could show little inhibitory effect like the TAR GUU CTE-Rz. By contrast, no ribozyme could inhibit the expression of a gene for Actin nonspecifically, indicating the CTE-linked ribozyme specificity.

Figure 7 shows efficient expression and intracellular localization of transcripts of tRNA promoter-linked ribozyme and CTE-linked ribozyme in LTR-Luc HeLa cells.

In order to determine the expression level of ribozymes and the intracellular localization of each ribozyme in LTR-Luc HeLa cells, Northern blotting analyses were carried out. Total RNA from LTR-Luc HeLa cells that had been transfected with each expression vector was separated into nuclear (N) and cytoplasmic (C) fractions. Levels of each transcribed enzyme were then examined by Northern blotting analysis with the probes (SEQ ID NOS: 12-15) specific for each transcript. Previously, the inventors confirmed that in all cases that the inventors examined, the transcript from the inventors' expression system under the control of the promoter of the human gene for tRNA^{Val} had been transported to the cytoplasm individually and in addition, no transcript had been detected in the nucleus (S. Koseki et al., 1999, J. Viol., 73:1868-1877). As shown in Fig. 7 and as expected from the properties of the inventors' tRNA^{Val} promoter-expression system, all tRNA^{Val} promoter-linked enzymes were found in the cytoplasmic fraction with a similar, significant extent and none was detected to any significant extent in the nuclear fraction. Both the ribozyme and the CTE-linked ribozyme were localized in the cytoplasm with a similar expression level, regardless of the presence or absence of the CTE sequence.

The CTE sequence is known to be a signal for the transport of D-type retrovirus RNA to the cytoplasm. Therefore, one might imagine that the increase in the inhibitory effect of ribozymes by connecting with CTE might have resulted from the transport of the ribozyme-transcript to the cytoplasm and by the co-localization of the ribozyme with target mRNA. However, it can be ignored that the influence of transport through the CTE-related pathway on the efficiency of the ribozyme, because, as far as the inventors' expression system is concerned, all transcripts could be transported to the cytoplasm individually. Moreover, there was no difference in the expression level of each enzyme. Therefore, an increase in the efficacy of a ribozyme by connecting with CTE should have resulted from other effects, most likely attributable to the function of RNA helicase A.

Figure 8 shows the dominant negative effect on the CTE-linked ribozyme. To investigate whether the increase in efficacy by CTE-ribozyme was caused by the linked CTE sequence, the inventors examined the dominant negative effect of CTE on the CTE-linked ribozyme. As can be seen from Figure 8, co-transfection of the TAR GUU Rz-expression vector together with the CTE-expression vector in trans did not affect the efficiency of the TAR GUU Rz (lanes 3 and 4). Only when the TAR GUU Rz was linked with CTE *in cis*, was a significant effect observed (lane 5). However, the increased efficiency of CTE-Rz was lost by the co-transfection of the CTE-Rz-expression vector together with the CTE-expression vector (lane 6). This dominant negative effect by CTE strongly supports the participation of *in cis* linked CTE in the improvement of the ribozyme-efficacy.

### Example 2: CTE linked ribozyme (II)

### Material and Methods:

### Construction of plasmids

The construction of ribozyme-expression vectors derived from the pUC-dt plasmid was described previously (Koseki, S. et al. (1999) J. Virol. 73, 1868-1877 ; Kuwabara, T. et al. (1999) Proc. Natl. Acad. Sci. USA 96, 1886-1891). To generate CTE-Rz-expression vectors, the present inventors inserted the CTE (SRV CTE-1; Tang, H. et al. (1997) Science 276, 1412-1415 ; GrŸter, P. et al. (1998) Mol. Cell 1, 649-659) sequence derived from the simian type D retrovirus (Fig. 2A). pUC-dt was double-digested by *Csp* 45I and *Sal* I and each ribozyme sequence, with *Kpn* I and *Eco*R V sites and the terminator sequence UUUUU at the 3' end, was cloned into this plasmid (Fig. 2A). The *Kpn* I and *Eco*R V sites were used for subsequent insertion of the CTE sequence. The pRcCMV-mychDbp5 vector encodes the gene for human Dbp5 helicase (hDbp5) with the myc tag at the N-terminus (Schmitt, C. et al. (1999) EMBO J. 18, 4332-4347). The pcDNA3 RHA-HA vector encodes the gene for human RNA helicase A with the HA tag at the N-terminus (Tang, H. et al. (1997) Science 276, 1412-1415 ; Li, J. et al. (1999) Proc. Natl. Acad. Sci. USA 96, 709-714).

### Measurement of activities of ribozymes in LTR-Luc HeLa cells

Luciferase activity was monitored basically as described elsewhere (Kuwabara, T. et al. (1999) Proc. Natl. Acad. Sci. USA 96, 1886-1891 ; Koseki, S. et al. (1998) Journal of Controlled Release 53, 159-173). LTR-Luc HeLa cells were plated at 80% confluence in twelve-well plates and incubated at 37°C in a CO₂ incubator. The cells were washed twice with phosphate-buffered saline (PBS) before (co)-transfection. Four µg of each ribozyme-expression plasmid and 150 ng of Tat-expressing plasmid (pCD-SRa/tat; Koseki, S. et al. (1998) Journal of Controlled Release 53, 159-173) were mixed with 4 µL of Lipofectin reagent (Gibco-BRL, Rockville, MD) in 400 µL of serum-reduced medium (OPTI-MEM I; Gibco-BRL), and incubated for 30 min at room temperature as described in the user's bulletin from Gibco-BRL. In the case of the assay of dominant negative activity, indicated amounts of plasmid were used for transfection (Fig. 3B). Then the mixture was gently added to cells. After 12 hrs, the medium was replaced by growth medium (DMEM) supplemented with 10% fetal calf serum and cells were cultured for another 24 hrs.

Luciferase activity was measured with a PicaGene kit (Toyo-inki, Tokyo, Japan) as described elsewhere (Kuwabara, T. et al. (1999) Proc. Natl. Acad. Sci. USA 96, 1886-1891 ; Koseki, S. et al. (1998) Journal of Controlled Release 53, 159-173). To normalize the efficiency of transfection by reference to β-galactosidase activity, cells were co-transfected with 50 ng of the pSV-β-Galactosidase control vector (Promega, Madison, WI) and the chemiluminescent signal due to β-galactosidase was determined with a luminescent β-galactosidase Genetic Reporter System (Clontech, Palo Alto, CA), as described elsewhere (Kuwabara, T. et al. (1999) Proc. Natl. Acad. Sci. USA 96, 1886-1891).

### Analysis of the cleavage activity of individual ribozymes in vitro

Each ribozyme, with or without the CTE sequence, and substrate RNA, the 5'-region of the LTR-Luc mRNA (300 nts) (Fig. 2B), were prepared *in vitro* using T7 RNA polymerase. Assays of ribozyme activity in vitro were performed, in 10 mM MgCl₂ and 50 mM Tris-HCl (pH 7.5) at 37 °C , under enzyme-saturating (single-turnover) conditions, as described elsewhere (Kuwabara, T. et al. (1998) Mol. Cell 2, 617-627 ; Kuwabara, T. et al. (1999) Proc. Natl. Acad. Sci. USA 96, 1886-1891). Each ribozyme (10 µM) was incubated with 2 nM 5'-32P-labeled substrate. The substrate and the products of each reaction were separated by electrophoresis on a 5% polyacrylamide/7 M urea denaturing gel and were detected by autoradiography.

### Western blotting analysis

NIH3T3 cells that had been transfected with each ribozyme-expression vector were harvested. Fifty µg of protein per lane were loaded on a 15% SDS-polyacrylamide gel. After electrophoresis, bands of protein were transferred to a polyvinylidene difluoride (PVDF) membrane (Amersham Co., Buckinghamshire, UK). The membrane was probed with a rabbit polyclonal antibodies against CPP32 and rabbit polyclonal antibodies against actin, as described elsewhere (Kuwabara, T. et al. (1998) Mol. Cell 2, 617-627). After incubation of the membrane with FITC-conjugated antibodies against IgG as second antibody, bands were detected with a Fluoro-Image Analyzer (Molecular Dynamics, Sunnyvale, CA). Blocking and detection were performed basically as described elsewhere (Kuwabara, T. et al. (1998) Mol. Cell 2, 617-627).

### Northern blotting analysis

Cytoplasmic RNA and nuclear RNA were isolated from LTR-Luc HeLa cells that had been transfected with individual ribozyme-expression vectors as described previously (Kuwabara, T. et al. (1998) Mol. Cell 2, 617-627 ; Koseki, S. et al. (1999) J. Virol. 73, 1868-1877 ; Kuwabara, T. et al. (1999) Proc. Natl. Acad. Sci. USA 96, 1886-1891). Thirty µg of total RNA per lane were loaded on a 3.0% NuSieve 3:1 agarose gel (FMC Inc., Rockland, ME). After electrophoresis, bands of RNA were transferred to a Hybond-N™ nylon membrane (Amersham Co.). The membrane was probed with synthetic oligonucleotides that were complementary to the sequences of the various ribozymes as described elsewhere (Koseki, S. et at. (1999) J. Virol. 73, 1868-1877). A synthetic probe complementary to the sequence of the CTE was used to determine the localization and the steady-state level of CTE RNA.

### Detection of interaction in cells between CTE-Rz and RNA helicases

Co-immunoprecipitation of CTE-Rz RNA and RNA helicase hDbp5 or RNA helicase A was done by transiently transfecting HeLa S3 cells with the CTE-Rz expressing vector and either pRcCMV-mychDbp5 (Schmitt, C. et al. (1999) EMBO J. 18, 4332-4347) or pcDNA3 RHA-HA (Tang, H. et al. (1997) Science 276, 1412-1415; Li, J. et al. (1999) Proc. Natl. Acad. Sci. USA 96, 709-714), basically as described elsewhere (Li, J. et al. (1999) Proc. Natl. Acad. Sci. USA 96, 709-714). Either c-myc-hDbp5 or HA-RNA helicase A was transiently co-transfected into HeLa S3 cells with the indicated versions of the TAR Rz4 expression vectors. Thirty-six hours after transfection, cell extracts were collected. Antibodies specific for either the c-myc-tag (Clontech Laboratories, Inc. Palo Alto, CA) or HA (hemagglutinin)-tag (Boehringer Mannheim GmbH, Mannheim, Germany) were used for the immunoprecipitations. Incubations were done overnight at 4°C with 10 µl of each antibody conjugated to 50 µl of protein A-agarose beads (Amersham Pharmacia, Uppsala, Sweden). The beads were washed three times with lysis buffer (50 mM Hepes-KOH, pH 7.5, 60 mM KCl, 2.5 mM EDTA and 0.1% Triton X-100). The bound RNA was extracted from beads by phenol extraction, ethanol precipitation, treatment with DNase I (3-h incubation at 37°C), phenol extraction and precipitation in ethanol and then quantitated. The extracted RNA was subjected to RT-PCR with the ribozyme-specific primer and products of PCR were visualized on agarose gels under UV light.

### Precipitation of RNA helicase hDbp5 and RNA helicase A that interact with in vitro synthesized CTE-Rz

Precipitation of proteins that interact with in vitro synthesized CTE-Rz was carried out basically as described elsewhere (Li, J. et al. (1999) Proc. Natl. Acad. Sci. USA 96, 709-714). Biotin-labeled RNA was synthesized with an AmpliScribe™ T7 transcription kit (Epicentre Technologies, Madison, WI). The molar ratio of Biotin-21-UTP (Clontech, Palo Alto, CA) to UTP in the reaction was 1 : 5. As a control, a biotinylated transcript without tRNA, Rz or CTE sequences (MCS; multi cloning site) was prepared using pBS (Stratagene, CA) as template and T7 RNA polymerase. 200 µL of cell extract from 2 x 10⁷ HeLa S3 cells transfected with either pRcCMV-mychDbp5 or pcDNA3 RHA-HA was mixed with 70 µg of biotinylated RNA, then incubated on ice for 10 min, and then adjusted to 1 ml with binding buffer. To this sample was added seventy microliters of streptavidin-conjugated agarose beads (Gibco BRL, Gaithersburg, MD) which were washed twice with binding buffer (20 mM Tris-HCl, pH 7.5, 60mM KCl, 2.5 mM EDTA and 0.1% Triton X-100) and suspended in 100 µL of binding buffer and kept on ice. After incubation overnight at 4°C, the beads were washed 3 times with washing buffer (20 mM Tris-HCl, pH 7.5, 350 mM KCl and 0.01% NP-40) and resuspended in 20 µL binding buffer. Proteins were eluted by boiling and separated by SDS-PAGE (7% polyacrylamide). For immunodetection of each RNA helicase, proteins were transferred to a PVDF membrane by the standard procedure, and then were probed with the antibodies mentioned above. As controls, whole cell lysates from HeLa cells transfected with pRcCMV-mychDbp5 or pcDNA3 RHA-HA were also subjected to Western blotting.

### Results:

Design of hybrid-ribozymes (CTE-Rz) and their effects on the expression of the LTR-luciferase chimeric gene. The CTE sequence was attached to the 3' end of a conventional tRNA-driven ribozyme (Fig. 2A, B). The present inventors quantitatively evaluated the intracellular activities of ribozymes (Rz) and CTE-ribozymes (CTE-Rz) directed against the TAR region of the LTR from HIV-1. This challenging target was chosen because of its extensive secondary structure (Fig. 3B). The target gene was stably expressed in HeLa cells and consisted of the long terminal repeat (LTR) of HIV-1 and a luciferase gene (Koseki, S. et al. (1998) Journal of Controlled Release 53, 159-173 ; Kuwabara, T. et al. (1999) Proc. Natl. Acad. Sci. USA 96, 1886-1891). Because this reporter is Tat-dependent, an expression plasmid for Tat was transiently transfected along with the ribozyme expression vectors.

To test the efficacy of the CTE-Rz design, the present inventors made five CTE-linked and non-linked ribozymes aimed at specific targets. Three ribozymes, namely, TAR Rz 1, LTR Rz 2 and Luc Rz 3, were designed to target relatively accessible sites located in predicted loop regions of the LTR-luciferase chimeric mRNA (Fig. 3B). As anticipated, these ribozymes significantly reduced expression of the reporter (Fig. 9, lanes 4, 6 and 8).

TAR Rz 4 (which is identical to TAR GUU Rz) and TAR Rz 5 were designed to target sites (Fig. 9, lanes 10 and 12) predicted, and later confirmed (see below), to be inaccessible within the well-documented stable stem structure of the TAR region. These ribozymes without the CTE were unable to affect luciferase reporter activity levels (Fig. 3B, lanes 10 and 12). When the CTE was attached, these ribozymes became remarkably efficient at catalyzing cleavage (Fig. 9, lanes 11 and 13), resulting in an 80% reduction in reporter activity. Furthermore, these CTE-coupled ribozymes reached levels better than those seen for the conventional ribozymes designed to cleave the open target site (TAR Rz 1, LTR Rz 2, Luc Rz 3). Attachment of CTE to other ribozymes also enhanced their activity: Importantly, TAR CTE-Rz 4 and TAR CTE-Rz 5 achieved suppression levels similar to those seen for TAR CTE-Rz 1, LTR CTE-Rz 2 and Luc CTE-Rz 3 (Fig. 9, lanes 5, 7, and 9). This suggests that addition of the CTE moiety enables all ribozymes to efficiently attack the chosen target site.

### Unwinding of the target RNA in vivo and the involvement of helicases

Because the stem of the TAR region must be unwound to permit cleavage and because the CTE was found to be required for cleavage, it follows that the CTE facilitates unwinding. It is also known that Tat and many other proteins bind the TAR region. That TAR CTE-Rz4 and TAR CTE-Rz5 had cleavage activity suggest that the presence of Tat was not interfering, perhaps because it was removed as part of the process of relieving the TAR secondary structure.

To demonstrate that the observed inhibitory effects were actually due to ribozyme-mediated cleavage, the present inventors tested an inactivated TAR Rz 4 that contains a single mutation in the catalytic site (Fig. 9, lanes 14 and 15). The lack of cleavage demonstrates that inhibition by TAR CTE-Rz4 is due to ribozyme-mediated cleavage, not antisense effects. Also, when fused to abundantly expressed tRNA, the CTE did not have a nonspecific effect on luciferase reporter activity (Fig. 9, lanes 3 and 15).

To verify the computer structure prediction that the TAR region is difficult to access *in vivo* because of RNA folding and not because of RNA-bound protein(s), *in vitro* cleavage assays were performed. Very little cleavage of the TAR substrate was observed for TAR Rz4 and TAR Rz5, either as conventional or CTE-fused ribozymes (data not shown), thereby suggesting that inherent folding of the TAR region is responsible for its inaccessibility. Furthermore, both conventional and CTE-bound versions of ribozymes (TAR Rz1, LTR Rz2 and Luc Rz3) directed against sites predicted to be accessible displayed similar significant *in vitro* activity levels (data not shown). Therefore, it seems that the CTE-mediated enhancement that occurs in cells is dependent on cellular factors, perhaps a helicase(s) that can resolve RNA secondary structure, as originally hypothesized.

### General applicability of hybrid-ribozymes

To test the general applicability of the CTE-ribozyme, the present inventors targeted endogenous mouse Procaspase-3 (CPP32) at five sites, including one predicted to be inaccessible (Fig. 10A; SEQ ID NOS:39-43 corresponding to Site 6 to Site 10, respectively). The nucleotide sequences of the ribozymes CPP Rz6, CPP Rz 7, CPP Rz8, CPP Rz9 and CPP Rz10 are shown in SEQ ID NOS:44 to 48, respectively. Mouse NIH3T3 cells were transfected with the ribozyme expression plasmids and Procaspase-3 expression levels were determined by Western blotting (Fig. 10B) and quantitated (Fig. 10C). Actin expression levels were used as controls. As seen previously for the LTR-luc reporter, CTE-linked riboyzmes were more effective than their conventional counterparts. In particular, CPP CTE-Rz 10 had a significant inhibitory effect (Fig. 10C, lane 13) whereas its parental ribozyme had virtually no effect (lane 12). None of the CTE-ribozymes interfered with actin expression. Similar results have been obtained for several other endogenous targets (data not shown).

Because these experiments used transient transfections (because transfection efficiency was not 100%), complete suppression was not observed. However, when the present inventors establish stable cell lines using a CTE-Rz, the present inventors achieve nearly 100% suppression of the targeted gene (data not shown). These results demonstrate the specificity, potent activity and general utility of CTE-ribozymes.

### The level of expression and localization of the hybrid-ribozyme

Ribozyme expression levels, stability, and localization are important determinants of ribozyme efficacy *in vivo* (Sullenger, B. A. & Cech, T. R. (1993) Science 262, 1566-1569; Koseki, S. et al. (1999) J. Virol. 73, 1868-1877; Kuwabara, T. et al. (1999) Proc. Natl. Acad. Sci. USA 96, 1886-1891). Since the CTE is known to be a signal for the transport of D-type retrovirus RNA to the cytoplasm (Tang, H. et al. (1997) Science 276, 1412-1415), it seemed possible that the CTE might enhance cytoplasmic transport of ribozymes, thereby leading to higher activities. Or, because the CTE has a stem structure, it might stabilize the ribozyme-containing transcript. To determine the cellular expression levels and localization of a CTE-ribozyme, the present inventors performed Northern blot analyses of RNA from fractionated cells transfected with TAR Rz4 and TAR CTE-Rz4 (Fig. 11A). In our previous studies, in all cases examined, conventional ribozymes with high activity under control of the human tRNA^{Val} promoter were found exclusively in the cytoplasm (C) (Kawasaki, H. et al. (1998) Nature 393, 284-289 ; Kuwabara, T. et al. (1998) Nature Biotechnol. 16, 961-965 ; Kuwabara, T. et al. (1998) Mol. Cell 2, 617-627). As anticipated, TAR Rz4 and TAR CTE-Rz4 were expressed at similar levels and fractionated to the cytoplasm, not to the nucleus (Fig. 11A). Therefore, CTE-mediated enhancement of ribozyme activity does not occur because of increased expression levels, either as a consequence of increased stability or transport efficiency.

### Importance of the CTE sequence for enhanced efficiency

Since the improved efficacy of the CTE-Rz appeared to be attributable to properties other than enhanced transcript stability and intracellular transport, it seemed likely that the ability of CTE to interact with a helicase(s) was responsible. To test for involvement of a helicase(s), the present inventors did competition experiments. Co-expressing TAR CTE-Rz4 with tRNA-CTE, which contains no ribozyme, reduced the efficacy of TAR CTE-Rz4 cleavage of the LTR-luc substrate (Fig. 11B, lanes 6 and 7). That excess tRNA-CTE (Fig. 11B, lane 7), and not excess tRNA alone (no ribozyme and no CTE) (Fig. 11B, lane 6), interferes with TAR CTE-Rz4 cleavage suggests that tRNA-CTE and TAR CTE-Rz4 were competing for some limiting factor(s).

In addition, when the wild-type CTE of TAR CTE-Rz 4 was replaced by either of two mutant forms of CTE, ΔCTE (Tang, H. et al. (1997) Science 276, 1412-1415 ; Li, J. et al. (1999) Proc. Natl. Acad. Sci. USA 96, 709-714) and M36CTE (GrŸter, P. et al. (1998) Mol. Cell 1, 649-659 ; Braun, I. C., Rohrbach, E., Schmitt, C. & Izaurralde, E. (1999) EMBO J. 18, 1953-1965), ribozyme enhancement activity was lost (Fig. 11B, lanes 8 and 9). Two known RNA helicases have been demonstrated to interact with CTE (Tang, H. et al. (1997) Science 276, 1412-1415). The ΔCTE mutant is known not to interact with RNA helicase A (Tang, H. et al. (1997) Science 276, 1412-1415). The results obtained with these CTE mutants underscore the importance of the CTE and strongly support the participation of RNA helicase(s) in the increased efficiency of CTE-Rz activity.

### Interaction in vivo between the hybrid-ribozyme and RNA helicases

To test directly if either of these does interact with our CTE-Rz, co-immunoprecipitations were done using hDbp5 and RNA helicase A. First, TAR CTE-Rz4 was co-transfected with either c-myc-tagged hDbp5 (Schmitt, C. et al. (1999) EMBO J. 18, 4332-4347) or HA-tagged RNA helicase A (Tang, H. et al. (1997) Science 276, 1412-1415) into HeLa cells. Then, cell lysates were subjected to immunoprecipitation using either c-myc or HA antibodies. The resulting precipitates were evaluated for the presence of TAR CTE-Rz4 by RT-PCR. TAR CTE-Rz4 was clearly found to be in the hDbp5 precipitate, thereby indicating that TAR CTE-Rz4 and hDbp5 interact *in vivo* (Fig. 12A). TAR CTE-Rz4 also associates with RNA helicase A, but this interaction appears to be weaker than that observed for hDbp5 (Fig. 12A). When TAR Rz4, TAR M36CTE-Rz4 and TAR ΔCTE-Rz4 are used, no interaction is observed with hDbp5 and RNA helicase A, thereby confirming that the interaction between TAR CTE-Rz4 and either of the helicases is dependent on the presence of a functional CTE.

The reciprocal precipitation confirmed these results. In this case, in vitro transcribed biotinylated ribozymes were mixed with cell lysates derived from HeLa cells transfected with either c-myc-hDbp5 (Schmitt, C. et al. (1999) EMBO J. 18, 4332-4347) or HA-RNA helicase A (Tang, H. et al. (1997) Science 276, 1412-1415). The four ribozyme constructs used were TAR Rz4, TAR CTE-Rz4, TAR M36CTE-Rz4 and TAR ΔCTE-Rz4. To control for nonspecific interactions, a biotinylated transcript without tRNA, Rz or CTE sequences was used (Fig. 12B, MCS (multi cloning site)). Proteins were precipitated using avidin-conjugated agarose beads which recognize biotin. The precipitates were probed for c-myc-hDbp5 or HA-RNA helicase A by Western blotting. Only TAR CTE-Rz4 was found to be complexed with c-myc-hDbp5 and HA-RNA helicase A, verifying that the CTE enhances CTE-Rz activity by preferentially interacting with hDbp5 and/or RNA helicase A.

### Discussion:

By adding the CTE sequence to our tRNA-Rz, the present inventors were able to efficiently cleave sites that had previously been refractory because of local RNA folding. The presence of the CTE also improved cleavage activity for ribozymes that were already functional. It is also noteworthy that TAR CTE-Rz4 and TAR CTE-Rz5 were able to cleave TAR even in the presence of Tat, a protein known to bind TAR. When CTE mutants defective for RNA helicase interaction were substituted for wild-type CTE, the resulting TAR ΔCTE-Rz4 and TAR M36CTE-Rz4 completely lost activity. Therefore, to be effective, the CTE must be present in cis and must retain the capacity to interact with an RNA helicase(s).

The increased activity mediated by the CTE does not appear to involve increased stability, expression or transport of the ribozyme. Rather, the CTE seems to mediate its effects by interacting with RNA helicases. Two RNA helicases (hDbp5 and RNA helicase A) have been demonstrated previously to interact with CTE. The present inventors demonstrated that our CTE-Rz indeed interacted with these RNA helicases (at least with RNA helicase hDbp5, and to a lesser extent, with RNA helicase A (Fig. 12A)) in mammalian cells. RNA helicase hDbp5 has been shown to interact with an adapter protein known as TAP (Tip-associated protein) (GrŸter, P. et al. (1998) Mol. Cell 1, 649-659). This interaction is crucial for hDbp5-CTE interaction. By using cells devoid of TAP (QCl-3 cells; Kang, Y. & Cullen, B. R. (1999) Genes Dev. 13, 1126-1139), the present inventors have obtained preliminary evidence indicating that TAP is essential for CTE-Rz activity (data not shown), thereby further implicating hDbp5 in CTE-Rz activity. Since RNA helicases, including hDbp5, have been shown to possess RNA unwinding activity (Lee, C.-G. et al. (1993) J. Biol. Chem. 268, 116822-16830 ; Wagner, J. D. O. et al. (1998) EMBO J. 17, 2926-2937 ), the present inventors hypothesize that the CTE is recruiting this helicase(s) to the target site where it unwinds inhibitory structures. It is attractive to consider that the helicase may even be able to slide the tRNA-Rz along a transcript, consistent with the sliding mechansims of action demonstrated for several RNA helicases (Fig. 6; Lee, C.-G. et al. (1993) J. Biol. Chem. 268, 116822-16830 ; Wagner, J. D. O. et al. (1998) EMBO J. 17, 2926-2937 ). The key aspect of our CTE-ribozyme is that it overcomes a major obstacle of ribozyme use, thereby greatly increasing the general utility of ribozymes. In particular, attachment of the CTE has made it possible to suppress expression of genes that were previously found to be recalcitrant to ribozyme cleavage.

Despite recent advances in ribozyme technology that generally improved the utility of ribozymes, ribozyme design continues to be problematic. For a ribozyme to be successful it must have an easily accessible target sequence. Until now, such a target site was identified based on computer-aided structural predictions of the target RNA or by unwidely trial-and-error experiments. To overcome this limitation the present inventors sought to construct a ribozyme that would be able to access any target site independent of its local secondary or tertiary structure. Hoping to take advantage of the natural ability of RNA helicases to modulate RNA structure, the present inventors decided to connect a ribozyme to an RNA helicase. This was accomplished by linking our ribozyme to a sequence, the constitutive transport element (CTE), that has been shown to interact with an RNA helicase(s).

For all mRNAs tested, our CTE-ribozymes suppressed expression much more efficiently than the parental, non-CTE ribozymes. Most importantly, they were able to cleave the target mRNA at any site, regardless of the predicted secondary or tertiary structure. All of the CTE-ribozymes displayed robust activity in cell culture and in many cases were able to work when the parental, non-CTE ribozymes were inactive. Therefore, the present inventors believe the CTE-ribozyme has broad applicability because it is extremely easy to design and use, whereas previous ribozyme technology required specialized skills.

### Example 3: Poly(A) linked ribozyme

### Materials and Methods

### Construction of vectors that encode poly(A)-linked ribozymes

The construction of ribozyme-expression vectors derived from plasmid pUC-dt was described previously (Koseki, S., *et al. J. Virol*. **73**, 1868-1877 (1999)). To generate poly(A)-linked Rz-expression vectors, the present inventors inserted a poly(A) sequence of 60 nucleotides (Fig. 13b). pUC-dt was double-digested with *Csp* 45I and *Sal* I and each individual ribozyme sequence, with *Kpn* I and *Eco*R V sites and the terminator sequence UUUUU at the 3' end, was cloned into the plasmid (Fig. 13b). The *Kpn* I and *Eco*R V sites were used for subsequent insertion of the poly(A) sequence.

### Culture and transfection of HeLa cells

HeLa cells were grown in Dulbecco's modified Eagle's medium (DMEM) supplemented with 10% FCS. HeLa cells that stably expressed the human Fas gene (HeLa-Fas cells) were prepared as described elsewhere (Wajant, H. *et al. Current Biology* **8**, 113-116 (1998)). Transfections were carried out with the Lipofectin™ reagent (GIBCO-BRL) as described elsewhere (Kawasaki, H., *et al. Nature* **393**, 284-289 (1998)). Each line of ribozyme-transfected HeLa-Fas cells was selected by incubation with G418 for three weeks.

### Analysis by RT-PCR

Total RNA was isolated from HeLa-Fas cells with Isogen^{TM} (Nippon Gene, Toyama, Japan) according to the manufacturer's protocol. RT-PCR was performed using an RNA PCR Kit ver. 2 (Takara, Kyoto, Japan) with FADD upstream (nt. 110-134) and downstream (nt. 589-610) primers or CBP upstream (nt. 442-467) and downstream (nt. 632-655) primers as a control. The products of PCR were analyzed by electrophoresis on a 2% agarose gel.

### Western blotting analysis

HeLa-Fas cells that had been transfected with individual ribozyme-expression vectors were harvested. Proteins were resolved by SDS-PAGE (5% or 10.0% polyacrylamide) and transferred to a PVDF membrane (Funakoshi Co., Tokyo, Japan) by electroblotting as described elsewhere (Kawasaki, H., *et al. Nature* **393**, 284-289 (1998)). Immune complexes were visualized with an Amplified AP-immunblot kit (Bio Rad) using specific antibodies against human FADD (Santa Cruz), human CBP (CREB binding protein; Santa Cruz), human FLASH (Santa Cruz), human Caspase 9 (Santa Cruz) or human PTEN (Santa Cruz).

### Biotin-labeled RNA "pull-down" assay in vitro

The association between a poly(A)-linked ribozyme and RNA helicase eIF4AI was detected by a biotin-labeled RNA "pull-down" assay *in vitro* as described elsewhere (Rodgers, J. T. *et al. Anal. Biochem.* **277**, 254-259 (2000) ; Li, J., *et al. Proc. Natl. Acad. Sci.* USA **96**, 709-714 (1999)). Each biotin-labeled ribozyme was synthesized with a Biotin RNA Labeling Mix kit (Boehringer Mannheim).

### Analysis by immunoprecipitation-RT-PCR (IP-RT-PCR)

The association *in vivo* between a poly(A)-linked ribozyme and RNA helicase eIF4AI was detected by IP-RT-PCR analysis as described elsewhere (Rodgers, J. T. *et al. Anal. Biochem*. **277**, 254-259 (2000) ; Li, J., *et al. Proc. Natl. Acad. Sci*. *USA* **96**, 709-714 (1999)).

### ELISA of helicase activity

Helicase activity of poly(A)-linked ribozyme-protein complexes was measured by an ELISA as described in Hsu, C. C. *et al. Biochem. Biophys. Res. Comm..* **253**, 594-599 (1998).

### Assays of the activites of a poly(A)-linked or -non-linked ribozyme-protein complexes in vitro

Poly(A)-linked or -non-linked ribozyme-protein complexes were described in the text. Partial mRNAs for FADD (nt. 60-134 and nt. 140-206 indicated, respectively, by green and purple lines in Fig. 1c) used as substrates were labeled with [α-³²P]-ATP by T4 polynucleotide kinase. Duplexes of substrates used in this assay were prepared by hybridizing the respective ³²P-labeled mRNAs as described in Hsu, C. C. *et al. Biochem. Biophys. Res. Comm..* **253**, 594-599 (1998). *In vitro* unwinding and cleavage assays by respective ribozymes were described elsewhere (Kuwabara, T., *et al. Mol. Cell* **2**, 617-627 (1998)).

### Detection of apoptosis

Percentages of apoptotic cells were determined by "TUNEL" analysis as described in Kawasaki, H., *et al. Nature* **393**, 284-289 (1998). Cells were fixed for 15 min in 4% paraformalaldehyde, permeabilized with 0.1% Triton X-100, washed with PBS and incubated in 1x terminal deoxy-nucleotidyltransferase (TdT) buffer that contained 300 U/ml TdT and 40 M biotin-dUTP for 60 mm at 37°C according to the protocol from the manufacturer of the TUNEL kit (Boehringer-Mannheim Mannheim, Germany). Cells were then washed with PBS. TUNEL-positive cells were detected by incubation with FITC-conjugated streptavidin for 30 min at 37 °C. Detection of apoptotic bodies by staining of DAPI was described elsewhere (Kawasaki, H., *et al. Nature* **393**, 284-289 (1998)).

### Screening of functional gene using the randomized Rz-A60 library

A randomized Rz-A60 library with ten randomized nucleotides in each substrate-binding arm was constructed using the retrovirus expression system (Kuwabara, T., *et al. Mol. Cell* **2**, 617-627 (1998)). After infection with the randomized Rz-A60 library expressing retrovirus, HeLa-Fas cells that expressed the randomized Rz-A60 were treated with the Fas specific antibody. A60 represents a poly(A) sequence consisting of 60 nucleotides. After 24 h, survived clones were picked up and their genomic DNAs were purified. Sequences of Rz-A60 were determined by the direct sequencing and the target genes of Rz-A60 were identified from the gene databases (BLAST search).

### Results:

The present inventors created new ribozymes by combining the cleavage activity of a hammerhaed ribozyme with the sliding and unwinding activity (Jankowsky, E. *et al. Nature* **403**, 447-451. (2000)) of the endogenous RNA helicase eIF4AI (Fig.13a). To connect the helicase to the ribozyme, the present inventors added a naturally occurring RNA motif, a poly(A) sequense to the 3 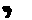 end of the ribozyme. This poly (A) sequence interacts with RNA helicase eIF4AI via interactions with poly(A)-binding protein (PABP) and PABP-interacting protein-1 (PIAP) (Craig, A. W. *et al. Nature* **392**, 520-523 (1998) ; Gallie, D. R. *Gene* **216**, 1-11. (1998) ; De Gregorio, E. *et al. EMBO J.* **18**, 4865-4874. (1999)).

To construct poly(A)-linked ribozymes, the present inventors attached a poly(A) sequence (60 nucleotides) to the 3' end of a tRNA^{Val}-driven ribozyme (Fig. 13b ; designated tRNA^{Val}-Rz-A60). The present inventors evaluated the intracellular activities of various ribozymes and poly(A)-linked ribozymes (Rz-A60) targeted to the mRNA for the pro-apototic factor FADD (Fas-Associated Death Domain protein) (Chinnaiyan, A. M. *et al. Cell* **81**, 505-512 (1995) ; Muzio, M. *et al. Cell* **85**, 817-27 (1996)). A diagram of the secondary structure of the 5' sequence of 300 nucleotides (nt.) of human FADD mRNA, as predicted by computer simulation with the MulFold program (Jaeger, J. A., Turner, D. H. and Zuker, M. *Methods in Enzymology* **183**, 281-306 (1989)), is shown in Figure 13c (SEQ ID NO:17). Successful inactivation by ribozymes of a specific gene *in vivo* normally depends on selection of an accessible target site. In order to test the efficacy of poly(A)-linked ribozymes, the present inventors designed four poly(A)-linked and -non-linked ribozymes aimed at specific targets. Three ribozymes, namely, FADD-Rz1, FADD-Rz2 and FADD-Rz3 (Fig. 13d), were designed to target inaccessible sites that are located within the stable stem-structure (Fig. 13c). By contrast, FADD-Rz4 (Fig. 13d) was designed, as a control, to target a relatively accessible site located in a loop region of the FADD mRNA (Fig. 13c). The present inventors cloned the various ribozymes, with and without a poly(A) sequence, into the parental tRNA^{Val}-expression vector, pUCdt, as indicated in Figure 13b.

To examine whether RNA helicase eIF4A would associate with tRNA^{Val}-Rz-A60 *in vitro*, the present inventors performed a biotin-streptavidin "pull-down" assay using biotin-labeled tRNA^{Val}-Rzs or tRNA^{Val}-Rz-A60s that had been transcribed by T7 polymerase *in vitro*. Extracts of HeLa cells that had been incubated with the biotin-labeled tRNA^{Val}-Rz or tRNA^{Val}-Rz-A60 were incubated with streptavidin beads. The beads were washed extensively and then bound proteins were eluted from beads. The eluted proteins were then analyzed by SDS-PAGE and Western blotting with eIF4AI-specific antibodies. The immunoblot indicated that tRNA^{Val}-Rz transcripts without a poly(A) sequence did not bind eIF4AI (Fig. 14a, lanes 2-5). By contrast, tRNA^{Val}-Rz-A60 transcripts did bound to eIF4AI (Fig. 14a, lanes 6-9), demonstrating the anticipated interaction between the tRNA^{Val}-Rz-A60 and the endogenous eIF4AI. As a negative control, the present inventors also tested poly(C)-linked tRNA^{Val}-Rz (tRNA^{Val}-Rz2-C60), which failed to bind to eIF4AI (Fig. 14a, lane 10). These results indicated that the endogenous RNA helicase eIF4AI associated with tRNA-Rz-A60 *in vitro*.

To confirm the interaction between the various forms of tRNA-Rz-A60 and the endogenous eIF4AI *in vivo*, the present inventors performed immunoprecipitation-RT-PCR (IP-RT-PCR). Plasmids encoding tRNA-Rz-A60 were used to transfect to HeLa cells. After 36 hours, eIF4AI- binding proteins and RNAs were precipitated with eIF4AI antibody-protein A-Sepharose beads. Then eIF4AI-binding RNAs were purified and subjected to analysis by RT-PCR with the appropriate ribozyme-specific primers. The present inventors first examined the levels of tRNA-Rz or tRNA-Rz-A60 transcripts in cells by RT-PCR. As shown in Figure 14b, the levels of expression were found to be nearly identical for each of the eight ribozymes (lanes 1-9), within the limits of experimental error. In the IP-RT-PCR analysis, eIF4AI interacted with all the tRNA-Rz-A60 transcripts (Fig. 14c, lanes 5-8). By contrast, the ribozymes without poly(A)-tails, such as the tRNA-Rz and tRNA-Rz-C60 transcripts, were not co-precipitated with eIF4AI (Fig. 14c, lanes 1-4, 9). These results demonstrate that eIF4AI interacts with tRNA-Rz-A60 *in vivo*.

To investigate whether the protein that binds to tRNA^{Val}-Rz-A60 has helicase activity, the present inventors performed an ELISA for RNA helicase activity (Hsu, C. C. *et al. Biochem. Biophys. Res. Comm.*. **253**, 594-599 (1998)). The present inventors first generated the sense strand, namely, biotin-labeled partial mRNA for human FADD (nt. 60-134; indicated by a green line in Fig. 13c), using biotin-UTP and T7 polymerase. Next, the corresponding DIG (digoxigenin)-labeled complementary strand, DIG-labeled partial FADD complementary RNA (nt.140-206; purple line), was transcribed by T7 polymerase with a DIG-UTP (Fig. 15a). These RNAs were allowed to hybridize with each other and were bound to wells of streptavidin-coated microtiter plates so that the helicase could catalyze the unwinding reaction. The present inventors isolated DIG-labeled tRNA-ribozyme-protein complexes for use as the source of potential helicase activity (Fig. 15a). If the substrate RNA were not unwound, the present inventors would expect the DIG-labeled FADD mRNA (nt. 140-206) to be retained on the plate and to be detecteable with DIG-specific antibodies (α-DIG) coupled to alkaline phosphatase (AP). Measurements of absorbance allowed determination of the efficiency of unwinding. As shown in Figure 15b, proteins that bound to tRNA^{Val} (control) and tRNA^{Val}-ribozymes did not have any unwinding activity, while the protein that bound to each tRNA^{Val}-Rz-A60 did have helicase activity. Furthermore, the protein that bound to tRNA^{Val}-Rz-C60 did not have any helicase activity. Therefore, these results suggest that the tRNA^{Val}-Rz-A60 complex slides along the substrate and unwinds the substrate as a consequence of an interaction with the endogenous RNA helicase eIF4AI. It is important to emphasize here that the interacting protein must have been the endogenous RNA helicase because the present inventors did not overexpress any helicases in our studies.

Moreover, to examine whether tRNA^{Val}-Rz-A60-protein complexes can cleave target inaccessible sites, the present inventors performed *in vitro* cleavage assay by these ribozyme-protein complexes. At first, the present inventors generated duplexes as substrates by hybridizing partial mRNAs for FADD (as those shown in Fig. 15a but without labeling with either DIG or biotin) and mixed with poly(A)-linked or -non-linked ribozyme-protein complexes as described above. As shown in Figure 15c, poly(A)-non-linked FADD-Rz1, -Rz2, -Rz3 and -Rz2-C60 did not unwind the duplexes (lanes 2, 4, 6 and 8) and, thus, they were unable to cleave the substrate. By contrast, FADD-Rz1-A60, -Rz2-A60 and -Rz3-A60 were clearly capable of unwinding and cleaving the substrate (lanes 3, 5 and 7). However, the inactive FADD-Rz2-A60 ("I-FADD-Rz2-A60", with a single G-to-A mutation at the catalytically important conserved nucleotide) could unwind duplexes but did not cleave the substrate (lane 9). These results clearly demonstrated that tRNA^{Val}-Rz-A60-protein complexes had two activities such as unwinding and cleavage *in vitro*. Thus, importantly, poly(A)-linked ribozymes could cleave inaccessible target sites that were not cleavable by conventional ribozymes.

To examine the effect of hybrid ribozymes targeted to the mRNA for the pro-apoptotic factor FADD, the present inventors used HeLa cells that expressed a gene for Fas. The present inventors examined the level of FADD mRNA in cells that expressed poly (A)-linked and -non-linked ribozymes by RT-PCR. As shown in Figure 16a, the level of FADD mRNA in HeLa cells that expressed a conventional ribozyme, FADD-Rz1, -Rz2 or -Rz3, was unchanged as compared with that of the FADD mRNA in untransfected (WT; wild type) HeLa cells (J. Exp. Med. 103:273 (1956)) (lanes 1, 2, 4 and 6). However, the level of FADD mRNA was reduced dramatically in HeLa cells that expressed a poly(A)-linked ribozyme, namely, FADD-Rz1-A60, -Rz2-A60 or -Rz3-A60, as compared with levels in WT HeLa cells and in cells that expressed FADD-Rz1, -Rz2 or -Rz3 (lanes 3, 5 and 7). When the present inventors targeted an accessible target site, poly(A)-non-linked FADD-Rz4 could also cleave the substrate: the level of FADD mRNA was reduced by both FADD-Rz4 and FADD-Rz4-A60 (lanes 8 and 9). Moreover, in order to demonstrate that the observed inhibitory effects were actually due to ribozyme-mediated cleavage, the present inventors used an inactive form of FADD Rz2-A60. Inactive Rz2-A60 (I-Rz2-A60; lane 10) had no effect on the level of FADD mRNA. These results clearly demonstrated that our poly(A)-linked ribozymes were very efficient at cleaving their target mRNA as a result of their association with the RNA helicase eIF4AI.

The present inventors next examined the level of FADD itself in HeLa cells that expressed poly (A)-linked or -non-linked ribozymes by the Western blotting. In HeLa cells that expressed FADD-Rz1-A60, -Rz2-A60 or -Rz3-A60, the level of FADD was significantly lower than that in WT HeLa cells or in cells that expressed FADD-Rz1, -Rz2 or -Rz3 (Fig. 16b, lanes 3, 5 and 7). The level of CBP protein (CREB binding protein), used as a control in the various lines of cells, remained the same as that in WT HeLa cells. These results show that, in agreement with the results of RT-PCR, the effects of Rz-A60 were significantly greater than those of the conventional parental ribozymes. Moreover, an inactive Rz2-A60 had no inhibitory effect (Fig 16b, lane 11). These results lend further support to our earlier conclusion that the inhibitory effects were due to ribozyme-mediated cleavage. Decreased levels of FADD reflected decreased levels of FADD mRNA.

Fas is a member of the family of receptors for tumor necrosis factor and it induces apoptosis when crosslinked with Fas-sepecific antibodies (Yonehara, S. *et al. J. Exp. Med.* **169**, 1747-1756 (1989) ; Trauth, B. C. *et al. Science* **245**, 301-305 (1989); Suda, T. *et al. Cell* **75**, 1169-1178 (1993)). Upon crosslinking with the antibodies, Fas induces formation of the death-inducing signaling complex, which consists of the adaptor molecules FADD and caspase 8. The resulting release of active caspase 8 initiates the apoptotic processes (Chinnaiyan, A. M. *et al. Cell* **81**, 505-512 (1995) ; Muzio, M. *et al. Cell* **85**, 817-27 (1996)). However, Fas-induced apoptosis only ensues after subsequent steps which commit the cells to an activation of an effector caspase. The full details of Fas-induced apoptosis mechanism remain unknown. Since the level of FADD was reduced in cells that expressed FADD-Rz-A60 but not FADD-Rz, the present inventors examined whether the phenotype of cells that expressed FADD-Rz-A60 might have changed. The present inventors examined the viability of cells that expressed various ribozymes during apoptosis induced by Fas-specific antibodies (α-Fas). The present inventors counted viable cells 24 h after the start of treatment with α-Fas.

As shown in Figure 16c, apoptosis occurred in wild-type cells after the treatment with α-Fas. By contrast, cells that expressed poly (A)-linked ribozymes, FADD-Rz-A60, did not undergo apoptosis. Cells expressing normal ribozymes underwent apoptosis, with the exception of cells that expressed FADD-Rz4, whose target site was accessible without unwinding (see also Fig. 16a, lane 8, and Fig. 16b, lane 8). Since the phenotype of cells that expressed FADD-Rz-A60 was same as that of cells that expressed FADD-Rz4, it seems likely that the poly(A) motif did not affect expression of any other genes. Similar results were obtained by staining *in situ* with 4',6-diamidino-2-phenylindole dihydrochloride n-hydrate (DAPI) (Fig. 16d). It is likely that FADD-Rz-A60 with high-level activity will be useful for future investigations of the details of the Fas-induced pathway to apoptosis.

The present inventors established a novel functional gene screening system for the signal pathway of Fas-induced apoptosis using the randomized Rz-A60 expression libraries. In this system, the present inventors randomized ten nucleotides in each substrate-binding arm of Rz-A60, and, then, HeLa-Fas cells were transduced by retroviral vectors that carried the randomized Rz-A60 expression libraries (Fig. 17a). After treatment of the randomized Rz-A60 introduced HeLa-Fas cells with the Fas specific antibodies, cells that survived were collected and a respective genomic DNA was isolated from each clone. Sequencing of the randomized region of Rzs-A60 in each genomic DNA enabled us to rapidly identify genes that are responsible in the apoptotic pathway (Fig. 17b). In this first screening, the present inventors identified many interesting genes that have pro-apoptotic functions, during the Fas-induced apoptosis signaling, such as human FLASH, human caspase 9, human FADD, and human PTEN (Fig. 17b), and the present inventors confirmed expression levels of these factors and their apoptotic functions by making specific Rzs or Rzs-A60 (Fig. 17c, d). The present inventors are presently analyzing other unknown genes (not yet deposited to Gene Banks) that were identified in this first screen to have pro-apoptotic functions. It should be mentioned that, although, theoretically, it is possible to make similar libraries with the conventional ribozymes without the poly(A)-tail, the level of false positives was reduced significantly by the use of randomized Rzs-A60 libraries (data not shown) since the hybrid ribozymes can attack any site within any mRNA. In accord with this notion, as demonstrated in Figure 17d, in the absence of the poly(A)-tail, the present inventors would not have identified FADD and PTEN (and other unknown genes) in our first screen with the similar libraries with the conventional ribozymes. This demonstrates the successful application of the hybrid ribozyme as a general method for gene discovery.

### Advantages of the Invention

Ribozymes are so called RNA restriction enzymes, which can cleave RNA site-specifically. Theoretically, ribozymes can be applied to any genes (mRNA). The applicability of ribozymes is immeasurable such that they can be applied to antiviral agents, oncogene expression inhibitors, specific gene expression inhibitors for functional analysis, and the like. Many researchers have tackled with the study on control of gene expression using ribozymes so far. The results of these studies have been gradually accumulated. Recently, papers reporting the success in inhibiting expression with ribozymes are increasingly published. However, the researchers cannot always succeed in inhibiting expression using ribozymes. The establishment of a more general technique using ribozymes (gene expression control technique using ribozymes) has been expected. The inventors had improved the expression system of ribozymes so tat they have succeeded in developing such a technique using ribozymes from which highly efficient inhibition of expression can be expected. Even such a technique has a drawback that the inhibitory effect of ribozymes are significantly affected by selection of cleavage sites and several ribozymes are required to be designed for one gene. Similar problems arise in the field of anti-sense. Development of techniques to select an effective Site of action is actively studied. The CTE-linked and poly(A)-linked ribozymes that the inventors have developed bind directly or indirectly to RNA helicase expressed in a cell. This binding allows functional nucleic acids, such as a ribozyme, to easily target RNA with high-order structure that they could have not targetted so far, suggesting that selection of cleavage sites will no longer affect expression controlled by ribozymes. By the use of this technique, increased success rate of technique using ribozymes can certainly be obtained and dramatically decreased time required to develop such ribozymes can be expected.

In the above examples CTE was used as an adaptor for ribozymes and RNA helicase. RNA helicase is a powerful candidate as a protein binding to this CTE. Other candidate protiens have also been reported (P. Gruter et al., (1998) Mol. Cell 1:649-659; I.C. Braun et at. (1999) EMBO J. 18:1953-1965; Y Kang and B. R. Cullen (1999) Genes Dev. 13:1126-1139). Such a candidate protein is also known to strongly bind to another RNA helicase in a cell (S. S. Tseng et al., (1998) EMBO J. 17:2651-2662; C.A. Snay-Hodge et al., (1998) EMBO J. 17:2663-1676). RNA motif capable of binding to RNA helicase is not limited to CTE and poly(A) (H.J. Liao et al., (1998) Proc. Natl. Acad. Sci. USA 95:8514-8519). Skilled person in the art can predict and construct a wide variety of combinations of ribozyme and RNA motif. Major tasks are how the function of RNA helicase can be added to ribozymes and their utility. The present invention is of much value because it can satisfy both tasks.

As a result of genetic analysis project, decoding of human gene is said to end by 2003. There is a need to investigate functions of each gene in order to utilize the resultant information. The chimeric molecules of the present invention, sliding ribozymes can cleave target mRNA very effectively in a cell. A target recognition sequence of this ribozyme is randomized so as to function in cell, cells expressing a desired phenotype are collected, and the target recognition sequences of ribozymes expressing in the cell are analyzed. Thus the relationship of the desired phenotype and the sequence of a gene cleaved intracellularly by the ribozyme is shown. This is a novel technique to screen genetic functions, a gene discovery system, and is enabled by the present invention.

In the above examples, chimeric molecules of CTE or poly(A) and ribozymes are explained, but the present invention is not limited to these specific molecules. As clearly understood from the above detailed explanation of the invention, it is contemplated that the present invention encompasses any other molecules capable of sliding, and any other chimeric molecules which comprise a region having binding affinity for this molecule or a molecule that forms a complex with the molecule, and any functional region.

It is contemplated that all publications cited herein are included herein by reference. The present invention is not limited to only the specific embodiments and is intended to include any modifications and variants as long as they are fallen into the scope and spirit of invention as claimed in the appended claims.

## Claims

1. A chimeric molecule comprising a region with binding affinity for a molecule capable of sliding, and any functional region.

2. A chimeric molecule comprising a molecule capable of sliding and any functional region.

3. A chimeric molecule comprising a region with binding affinity for a molecule forming a complex with a molecule capable of sliding, and any functional region.

4. The chimeric molecule of claim 3 wherein said molecule forming a complex with a molecule capable of sliding is an adapter.

5. The chimeric molecule of any one of claims 1-3 which is a nucleic acid, a peptide nucleic acid, a protein or a combinat on thereof.

6. The chimeric molecule of claim 1 or 3 wherein said region with binding affinity for a molecule capable of sliding or said region with binding affinity for a molecule forming a complex with a molecule capable of sliding is a nucleic acid.

7. The chimeric molecule of any one of claims 1-3 wherein said molecule capable of sliding is a protein.

8. The chimeric molecule of claim 7 wherein said protein is selected from a helicase, a restriction enzyme, a polymerase and a repressor.

9. The chimeric molecule of any one of claims 1-3 wherein said functional region has enzyme or catalytic function, or inhibitory function or promoting function.

10. The chimeric molecule of any one of claims 1-3 wherein said functional region consists of a nucleic acid.

11. A chimeric molecule comprising a protein capable of sliding and any functional nucleic acid.

12. A chimeric molecule comprising a nucleic acid with binding affinity for a protein capable of sliding or a nucleic acid with binding affinity for a molecule forming a complex with said protein, and any functional nucleic acid.

13. The chimeric molecule of claim 12 wherein said nucleic acid with binding affinity for a protein capable of sliding or said nucleic acid with binding affinity for a molecule forming the complex with said protein binds directly or indirectly to said functional nucleic acid.

14. The chimeric molecule of claim 11 or 12 wherein said functional nucleic acid is selected from the group consisting of a ribozyme, a DNA enzyme, an antisense RNA, an antisense DNA and an aptamer.

15. The chimeric molecule of claim 14 wherein said ribozyme is a hammerhead ribozyme.

16. The chimeric molecule of claim 14 or 15 wherein the substrate-binding site of said ribozyme, antisense RNA or antisense DNA is randomized.

17. The chimeric molecule of claim 16 wherein the substrate-binding sites of hammerhead ribozyme, stem I region and stem III region are randomized.

18. The chimeric molecule of claim 11 or 12 wherein said protein capable of sliding is helicase.

19. The chimeric molecule of claim 11 or 12 wherein said nucleic acid with binding affinity for a protein capable of sliding or said nucleic acid with binding affinity for a molecule forming a complex with said protein is CTE (constitutive transport element) or a nucleic acid having substantially equivalent functions to said CTE, or a nucleic acid having poly(A) sequence.

20. The chimeric molecule of claim 19 wherein said nucleic acid having substantially equivalent functions to said CTE is artificially synthesized RNA or DNA.

21. The chimeric molecule of claim 19 wherein said CTE comprises a sequence shown in SEQ ID NO: 1 or a variant thereof having substantially equivalent functions to native CTE.

22. An expression vector comprising the chimeric molecule of any one of claims 1-3, 11, 12 or DNA encoding said chimeric molecule.

23. The expression vector of claim 22 wherein said chimeric molecule or DNA encoding said chimeric molecule is controlled by a promoter.

24. The expression vector of claim 23 wherein said promoter is a polymerase III promoter.

25. The expression vector of claim 24 wherein said polymerase III promoter is a tRNA promoter.

26. The expression vector of claim 25 wherein said tRNA promoter is a tRNA^{val} promoter or a variant thereof.

27. The expression vector of claim 22 further comprising a terminator sequence downstream of said chimeric molecule or DNA encoding said chimeric molecule.

28. The expression vector of claim 22 wherein said chimeric molecule comprises a functional RNA sequence and a CTE sequence.

29. The expression vector of claim 28 wherein said functional RNA sequence is selected from the group consisting of a ribozyme sequence, an antisense RNA sequence and an aptamer sequence.

30. The expression vector of claim 28 wherein said functional RNA sequence is a ribozyme sequence.

31. A method of producing the chimeric molecule of claim 22 comprising synthesizing RNA by a conventional method using the expression vector DNA of any one of claims 22-30 as a template and collecting the generated RNA.

32. A complex of the chimeric molecule of any one of claims 1-3, 11, 12 and a molecule capable of sliding.

33. The complex of claim 32 wherein said molecule capable of sliding is helicase.

34. The complex of claim 33 wherein said chimeric molecule binds to said helicase via an adapter.

35. A pharmaceutical composition comprising the chimeric molecule of any one of claims 1-3, 11, 12 as an active ingredient.

36. A pharmaceutical composition comprising the expression vector of claim 22 as an active ingredient.

37. A pharmaceutical composition comprising the complex of claim 32 as an active ingredient.

38. The pharmaceutical composition of any one of claims 35-37 which is used for preventing or treating viral diseases, diseases associated with apoptosis or diseases associated with abnormal gene expression.

39. A method of specifically cleaving a target nucleic acid using the chimeric molecule of any one of claims 1-3, 11, 12, the expression vector of claim 22, or the complex of claim 32.

40. The method of claim 39 wherein said target nucleic acid is a viral gene, protooncogene or a gene associated with apoptosis.

41. A method of specifically inhibiting or controlling a biological function of a target nucleic acid, using the chimeric molecule of any one of claims 1-3, 11, 12, the expression vector of claim 22, or the complex of claim 32.

42. Use of the chimeric molecule of any one of claims 1-3, 11, 12, the expression vector of claim 22, or the complex of claim 32, to clarify a biological function of a target nucleic acid.

43. A method for clarifying a biological function of a target nucleic acid comprising; specifically cleaving the target nucleic acid or specifically inhibiting the biological function of the target nucleic acid using the chimeric molecule of any one of claims 1-3, 11, 12, the expression vector of claim 22, or the complex of claim 32; determining a sequence of the cleavage site of said nucleic acid and the neighborhoods as needed; and examining the influence of the cleavage or the inhibition on biological activity.

44. The method of claim 43 wherein said functional nucleic acid is a ribozyme, an antisense RNA or an antisense DNA.

45. The method of claim 44 wherein said ribozyme is a hammerhead ribozyme.

46. The method of claim 44 or 45 wherein the substrate-binding site of said functional nucleic acid is randomized.

47. The method of claim 46 wherein said substrate-binding sites of hammerhead ribozyme, stem I region and stem III region are randomized.
